# EUROPEAN PATENT APPLICATION

(11) **EP 3 932 937 A1**
(43) Date of publication of application: **05.01.2022**
(21) Application number: 20184100.4
(22) Date of filing: 03.07.2020
(51) Int. Cl.: C07K 14/255, C07K 14/82, C12N 9/16

(54) **NOVEL PROTEIN TRANSLOCATION DOMAIN**

(71) Applicant: Universitätsklinikum Hamburg-Eppendorf, 20246 Hamburg (DE)
(72) Inventor: MARLOVITS, Thomas, 1170 Wien (AT); GÖSSWEINER-MOHR, Nikolaus, 4203 Altenberg bei Linz (AT); CHABLOZ, Antoine, 1053 Cugy (CH)
(74) Representative: Uexküll & Stolberg

(57) **Abstract**

The present invention relates to the field of protein delivery into eukaryotic cells. More particularly, the invention relates to a fusion protein comprising (i) a fragment of the *Salmonella* effector protein SptP and (ii) a protein of interest, such as a therapeutically active protein, which is to be introduced into a eukaryotic cell. The invention also relates to a method of introducing a protein of interest, such as a therapeutically active protein, into a eukaryotic cell. The invention also relates to the use of a fragment of the *Salmonella* effector protein SptP for introducing a protein of interest, such as a therapeutically active protein, into a eukaryotic cell.

## Description

The present invention relates to the field of protein delivery into eukaryotic cells. More particularly, the invention relates to a fusion protein comprising (i) a fragment of the *Salmonella* effector protein SptP and (ii) a protein of interest, such as a therapeutically active protein, which is to be introduced into a eukaryotic cell. The invention also relates to a method of introducing a protein of interest, such as a therapeutically active protein, into a eukaryotic cell. The invention also relates to the use of a fragment of the *Salmonella* effector protein SptP for introducing a protein of interest, such as a therapeutically active protein, into a eukaryotic cell.

### BACKGROUND OF THE INVENTION

Eukaryotic membranes are essential for cell homeostasis and therefore act as major barriers for macromolecules. Due to these fundamental functions of cell membranes, it is challenging to efficiently deliver proteins across these membranes to the cytosol of eukaryotic cells. Most of the currently attempted cytosolic protein delivery systems rely on polycations (cell penetrating peptides (CPP)^{1,2} or supercharged proteins), which are believed to mainly work through osmotic lysis of endosomes, which however is an inefficient process and requires rather high concentrations³. Nanoparticles⁴ have also been used, employing a variety of mechanisms depending on their compositions. In addition, bacterial toxins⁵⁻⁷ have the capability to translocate proteins through defined cell membrane pores. Finally, certain pathogenic bacteria have developed molecular machines allowing the transport of specific proteins across eukaryotic plasma membranes in order to manipulate eukaryotic cells^{8,9}.

In contrast to small molecules, which usually require extensive medicinal chemistry to achieve and demonstrate specificity, recombinant binding proteins such as Designed Ankyrin Repeat Proteins (DARPins)¹⁰, nanobodies¹¹ or monobodies¹² can readily be selected for high affinities and specificities against almost any target. However, these macromolecules cannot readily access the cytosolic space, which limits their utility for fundamental mechanistic studies and their analytical and therapeutic potential. Recombinant binders have previously been delivered, with various degrees of success, to intracellular compartments, using CPP^{13,14}, nanoparticles¹⁵, bacterial toxins¹⁶⁻¹⁸ and T3SS¹⁹⁻²³, with marked differences in cytosolic delivery efficiencies between cell types⁷. In addition, to our knowledge, no study has been published to rigorously verify the functionality of delivered DARPins, once they have reached the cytosol. For other recombinant binding proteins with inhibitory function against essential pathways, only two reports have described delivery of binders via a toxin system^{16,17}, while another study reported some activity of a binder coupled to a polyarginine tail¹⁴. Because of the limitations in cytosolic deliveries, the functionality of such synthetic proteins was mainly studied via transfection, stable intracellular expression or microinjection. For example, the RAS pathway was studied with multiple DARPins and a monobody targeting the RAS protein as well as DARPins targeting upstream (ErbB) or downstream (ERK) effector proteins²⁴⁻²⁹.

It was well known in the art that the N-terminal domain of the *Salmonella* effector protein SptP can be used for introducing a protein of interest into a eukaryotic cell via a bacterial type III secretion system (T3SS), preferably the T3SS of *Salmonella typhimurium.* It has been described that at least the 167 amino acids of SptP are required for translocation and protein delivery as both a secretion signal and a chaperone binding domain (CBD)³¹ are located in this domain. The present invention is based on the insight that less than 167 amino acids of SptP are required to effect protein translocation. In addition, it was observed that when using a shorted fragment of the SptP N-terminus fused to a protein to be delivered, the fusion protein enters the cell and becomes localized in the cytosol of the eukaryotic cell. This is in contrast with results obtained with fusion proteins comprising the 167 N-terminal amino acids of SptP. The latter become localized at the plasma membrane after cell entry.

### DESCRIPTION OF THE INVENTION

The present invention therefore refers to fusion proteins of the N-terminal domain of the effector protein SptP, which are shorter in length than those reported so far in the art and which still act as a protein translocation domain, and a protein or a protein domain of interest for which introduction into the cell is desired. Thus, the invention refers to a fusion protein, comprising
(a) a protein translocation domain consisting of amino acids 1-165 of the *Salmonella* protein SptP or a fragment thereof; and
(b) a protein or protein domain for delivery into a eukaryotic cell.

It is preferred that protein translocation domain of the fusion protein of the invention is derived from the SptP protein of *S*. *typhimurium.* The sequence of the SptP protein of *S*. *typhimurium* is depicted herein in SEQ ID NO:1. Accordingly, in one embodiment, the protein translocation domain consists of amino acids 1-165 of the N-terminal domain of the *Salmonella* protein SptP or a fragment of the sequence covering amino acids 1-165. This means that the protein translocation domain contains no sequence which would correspond to a part of the SptP protein of *S. typhimurium* beyond amino acid 165. Stated differently, the protein translocation domain does not contain any sequence corresponding to amino acids 166-543 of the SptP protein of *S. typhimurium.*

In a preferred embodiment, the protein translocation domain of the fusion protein of the invention consists of amino acids 1-165 of SEQ ID NO:1 or a fragment of such a sequence. As used herein, a fragment of the protein translocation domain consisting of amino acids 1-165 of SEQ ID NO:1 is a sequence in which one more amino acids are deleted at the N-terminus and/or the C-terminus of the protein translocation domain. Preferably such fragment comprises or consists of amino acids 1-100, amino acids 1-110, amino acids 1-120, amino acids 1-130, amino acids 1-140, amino acids 1-150 or amino acids 1-160 of the sequence of SEQ ID NO:1 (or an amino acid sequence having at least 90% identity to SEQ ID NO:1). In another embodiment, the fragment comprises or consists of amino acids 5-100, amino acids 5-110, amino acids 5-120, amino acids 5-130, amino acids 5-140, amino acids 5-150 or amino acids 5-160 of the sequence of SEQ ID NO:1 (or an amino acid sequence having at least 90% identity to SEQ ID NO:1). In yet another embodiment, the fragment comprises or consists of amino acids 10-100, amino acids 10-110, amino acids 10-120, amino acids 10-130, amino acids 10-140, amino acids 10-150 or amino acids 10-160 of the sequence of SEQ ID NO:1 (or an amino acid sequence having at least 90% identity to SEQ ID NO:1. In yet another embodiment, the fragment comprises or consists of amino acids 15-100, amino acids 15-110, amino acids 15-120, amino acids 15-130, amino acids 15-140, amino acids 15-150 or amino acids 15-160 of the sequence of SEQ ID NO:1 (or an amino acid sequence having at least 90% identity to SEQ ID NO:1. In yet another embodiment, the fragment comprises or consists of amino acids 20-100, amino acids 20-110, amino acids 20-120, amino acids 20-130, amino acids 20-140, amino acids 20-150 or amino acids 20-160 of the sequence of SEQ ID NO:1 (or an amino acid sequence having at least 90% identity to SEQ ID NO:1. In yet another embodiment, the protein translocation domain of the fusion protein of the invention comprises or consists of amino acids 25-100, amino acids 25-110, amino acids 25-120, amino acids 25-130, amino acids 25-140, amino acids 25-150 or amino acids 25-160 of the sequence of SEQ ID NO:1 (or an amino acid sequence having at least 90% identity to SEQ ID NO:1.

In a most preferred embodiment, the protein translocation domain of the fusion protein of the invention consists of amino acids 1-120, amino acids 1-121, amino acids 1-122, amino acids 1-123, amino acids 1-124, amino acids 1-125, amino acids 1-126, amino acids 1-127, amino acids 1-128, amino acids 1-129, amino acids 1-130, amino acids 1-131, amino acids 1-132, amino acids 1-133, amino acids 1-134, amino acids 1-135, amino acids 1-136, amino acids 1-137, amino acids 1-138, amino acids 1-139, amino acids 1-140, amino acids 1-141,amino acids 1-142, amino acids 1-143, amino acids 1-144, amino acids 1-145, amino acids 1-146, amino acids 1-147, amino acids 1-148, amino acids 1-149, amino acids 1-150, amino acids 1-151, amino acids 1-152, amino acids 1-153, amino acids 1-154, amino acids 1-155, amino acids 1-156, amino acids 1-157, amino acids 1-158, amino acids 1-159, or amino acids 1-160 of SEQ ID NO:1

While it is particularly preferred herein that the protein translocation domain comprise the N-terminal amino acid sequence of the SptP protein of *S. typhimurium* set out in SEQ ID NO:1, the invention also covers variants of this protein having at least 90% identity thereto. As used herein, variants of the protein of SEQ ID NO:1 are proteins with an amino acid sequence that differs from the amino acid sequence of SEQ ID NO:1 by one or more amino acid substitutions, additions or deletions. In general, any amino acid residue of the sequence shown in SEQ ID NO:1 may be substituted by another amino acid, provided that the resulting protein variant still effects delivery to a cell.

It is preferred that the substitutions are conservative substitutions, i.e. substitutions of an amino acid residue by another amino acid of a similar polarity, which acts as a functional equivalent. Preferably, the amino acid residue used as a substitute is selected from the same group of amino acids as the amino acid residue to be substituted. For example, a hydrophobic residue can be substituted with another hydrophobic residue, or a polar residue can be substituted with another polar residue having the same charge. Functionally homologous amino acids which may be used for a conservative substitution comprise, for example, non-polar amino acids such as glycine, valine, alanine, isoleucine, leucine, methionine, proline, phenylalanine, and tryptophan. Examples of uncharged polar amino acids comprise serine, threonine, glutamine, asparagine, tyrosine and cysteine. Examples of charged polar (basic) amino acids comprise histidine, arginine and lysine. Examples of charged polar (acidic) amino acids comprise aspartic acid and glutamic acid.

Also comprised by the term variant are protein sequences which include more amino acids than the sequence of SEQ ID NO:1, i.e. protein sequences in which one or more amino acids have been inserted. Such insertions may in principle occur at any position of the protein of SEQ ID NO:1. The insertions may be stretches of contiguous amino acids comprising, for example, 2, 3, 4, 5, 6, 7, 8, 9 or ten amino acids. Similarly, the term variant also includes protein sequences in which one or more amino acids are deleted as compared to the protein of SEQ ID NO:1. The deletion can involve several contiguous amino acid residues of the protein of SEQ ID NO:1, for example, 2, 3, 4, 5, 6, 7, 8, 9 or ten amino acids. Variants of the protein of SEQ ID NO:1 may also include structural modifications, for example modified amino acids, such as amino acids that have been altered by phosphorylation, glycosylation, acetylation, thiolation, branching and/or cyclization.

The variants of the SptP protein of SEQ ID NO:1, which have been modified by substitution, addition or deletion of amino acids, normally show a considerable degree of amino acid sequence identity to the SptP protein of SEQ ID NO:1. The identity on the amino acid level is at least 90%, 91%, 92%, 93%, 94%, 95%, 96%, 97%, 98% or 99% if the sequence of the variant is optimally aligned with that of SEQ ID NO:1. Methods and computer programs for determining amino acid homology are well known in the art.

In one embodiment, the fusion protein further comprises a linker between the protein translocation domain and the protein or protein domain that is to be delivered to the eukaryotic cell. The linker may comprise any number of amino acids as long as the activity of the protein translocation domain is maintained and the introduction of the fusion protein into the cell is still effected. The linker normally comprises 5-30 amino acids, preferably 8-25 amino acids, and more preferably 10-20 amino acids. The linker regularly includes several glycine residues, since this amino acid is less likely to affect protein folding of the active portion of the fusion protein. The fusion protein may also comprise additional components, for example, an affinity tag which facilitates binding of the fusion protein via a compound exhibiting binding affinity to the tag. For example, the affinity tag may be a poly-histidine tag comprising 6-12 histidine residues which specifically interacts with a nickel ion chelate matrix.

The protein or protein domain that is to be delivered to the eukaryotic cell preferably is a target-binding protein, i.e. a protein having affinity for a target molecule inside the cell. Preferably, the target molecule itself is a protein, such as a receptor protein. The protein or protein domain having affinity for a target molecule inside the cell preferably is an antibody or antibody fragment, a monobody or a monobody fragment, or a nanobody or a nanobody fragment. A suitable nanobody for use in the fusion protein of the invention is set forth herein as SEQ NO:4. Suitable monobodies for use in the fusion protein of the invention are set forth herein as SEQ NO:5-6. As used herein, the fusion protein can also comprise more than one protein or protein domain.

In another embodiment of the invention, the protein or protein domain that is to be delivered to the eukaryotic cell is an immunoregulatory protein, and in particular a cytokine, such as an interleukin, an interferon, a lymphokine, a tumor necrosis factor (TNF). More preferably, the the protein or protein domain that is to be delivered to the eukaryotic cell is derived from an interleukin selected from the group consisting of IL-2, IL-4, IL-6, IL-10, IL-11, IL-12, IL-13, and IL-23.

In another embodiment of the invention, the protein or protein domain that is to be delivered to the eukaryotic cell is a designed Ankyrin Repeat Protein (DARPin) or a toxic protein.

In another aspect, the protein or protein domain that is to be delivered to the eukaryotic cell is an antigenic structure that is introduced into a tissue for producing an immune response. Such an embodiment can be useful for preparing vaccines.

In yet another aspect the fusion protein defined hereinabove is for use in medicine, i.e. for introducing a therapeutically effective protein into a cell or tissue.

In yet another aspect the invention relates to a pharmaceutical composition comprising a fusion protein defined hereinabove. The pharmaceutical composition of the present invention normally comprises a physiologically acceptable carrier together with the fusion protein dissolved or dispersed therein as an active ingredient. Pharmaceutically acceptable carriers comprise, for example, water, saline, Ringer's Solutions, or dextrose solution. Further suitable carriers for compositions comprising the fusion protein of the invention are described in standard textbooks, for example, in "Remington's Pharmaceutical Sciences", Mack Pub. Co., New Jersey (1991). In addition to a carrier, the pharmaceutical composition of the invention may also comprise wetting agents, buffering agents, stabilizers, dyes, preservatives and the like in any concentration, provided that these compounds do not interfere with the activity of the fusion protein of the invention.

In a further aspect, the invention relates to a polynucleotide encoding a fusion protein as defined above. Polynucleotides which encode the fusion proteins of the invention may consist of DNA or RNA. The polynucleotide encoding the fusion protein of the invention refers to a DNA or RNA molecule which directs the expression of the desired fusion protein. The polynucleotides encoding the fusion protein of the invention may be cloned in an expression vector in order to provide means for recombinant preparation the fusion protein. Generally, expression vectors are self-replicating DNA or RNA constructs into which a polynucleotide of interest is inserted such that the coding sequence of the polynucleotide is operably linked to suitable regulatory elements which allow for the controlled expression of the coding sequence in a host cell. The specific control elements which are required for expression of the polynucleotide will depend on the host cell that is used and may include a transcriptional promoter, an operator, an enhancer to increase the mRNA expression level, a ribosome binding site, and suitable transcription and translation terminators. The promoter can be a constitutive or inducible promoter. Expression vectors may also contain an origin of replication for the autonomous replication of the vector within a host cell, and one or more selection marker which allow monitoring the transfection into the host cell.
The polynucleotide encoding a fusion protein can also be a bacterial or eukaryotic chromosome.

The expression vector may be designed for expressing the recombinant protein in a prokaryotic or eukaryotic host. Preferably, the expression vector will have the capability to stably replicate within the host cell so that the number of the polynucleotide which encodes the fusion protein of interest is increased within the cell. It is, however, also possible to use an expression vector which is unable to replicate within the cells and allows only transient expression of the fusion protein. Alternatively, expression vectors may be used which integrate into the genome of the host cell that is transduced or transfected with the expression vector. Various expression vectors are known in the prior art which are suitable for mediating expression of the fusion protein of the invention both in prokaryotic or eukaryotic host cells. Methods for introducing the expression vector into a host cell have also been extensively discussed in the literature. A variety of different methods may be used for transducing or transfecting the expression vector of the invention into a host cell, for example, electroporation, microinjection, transformation, transfection, protoplast fusion, microprojectile bombardment and the like. An exemplary vector for use in the present invention is the pCASP vector described by Widmaier et al. (2009). A modified version of this vector is set forth herein as SEQ ID NO:3.

In another aspect, the invention also relates to a host cell which comprises an expression vector harboring a polynucleotide encoding the fusion protein of the invention. The cells may be prokaryotes or eukaryotes. Suitable prokaryotes which can be transduced or transfected with an expression vector encoding the fusion protein of the invention include, for example, bacteria like Bacillus subtilis, Escherichia coli, and the like. Preferably, the prokaryotes which are transduced or transfected with an expression vector encoding the fusion protein of the invention are bacteria of the genus Salmonella, more preferably *S. typhimurium.* Even more preferably, the prokaryotes which are transduced or transfected with an expression vector encoding the fusion protein of the invention are non-virulent strains of S. typhimurium, such as ASB2519. Eukaryotes that may be used in the methods of the present invention include yeasts, in particular yeasts of the genus Saccharomyces, Pichia, or Dictyostelium. Higher eukaryotes include animal cell lines, in particular mammalian cell lines, for example cell lines derived from rodents, primates and human. Useful cell lines for use according to the invention include Chinese hamster ovary (CHO) cell lines, baby rat kidney (BRK) cell lines, Madin Darby canine kidney (MDCK) cell lines, NSO myeloma cells, monkey kidney COS cells, human embryonic kidney 293 cells, and cancer cell lines such as SKBR3 cells or HeLa cells.

The host cells of the invention which comprise a polynucleotide encoding the fusion protein of the invention, preferably in the form of an expression vector suitable of directing expression in the respective host cell, may be conveniently used for preparing the fusion protein. Preferably, the expression vector is introduced into the host cell such that the expression of the fusion protein is effected by propagation of the host cells. Accordingly, the invention also provides a method for preparing a fusion protein as defined herein, comprising the culturing of a host cell which comprises the polynucleotide encoding the fusion protein or the expression vector that includes the polynucleotide under conditions which allow for the expression of the fusion protein.

The invention also relates to a method of introducing a protein of interest, such as a therapeutically active protein, into a eukaryotic cell, comprising contacting the eukaryotic cell with a fusion protein as defined above. For example, the fusion protein can be introduced into the eukaryotic cell by means of a T3SS. Alternatively, the fusion protein can be introduced into the eukaryotic cell by means of liposome particles comprising the fusion protein.

In a final aspect, the invention relates to the use of a protein translocation domain consisting of amino acids 1-165 of the *Salmonella* protein SptP or a fragment thereof for introducing a protein of interest, such as a therapeutically active protein, into a eukaryotic cell. The protein translocation domain is composed as outlined hereinabove.

### BRIEF DESCRIPTION OF THE FIGURES

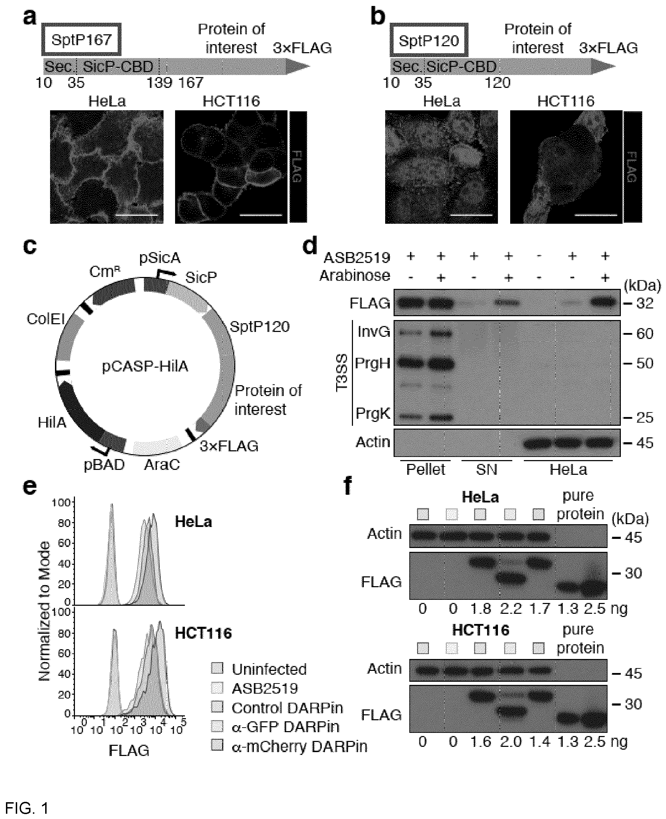

Figure 1 shows the approach for engineering an efficient cytosolic delivery system. a,b, (Top) Schematic of the transferred SptP167 and SptP120 3×FLAG tagged fusion recombinant proteins. Secretion signal (Sec.), SicP-chaperone binding domain (SicP-CBD) and amino acid numbers that mark the beginning and end of each SptP domain are indicated³¹. Representative anti-FLAG-immunostaining images of α-GFP FLAG-tagged DARPins transferred into HeLa and HCT116 cells using the SptP167 (**a**) or SptP120 (**b**) secretion tags (1 hour infection at a MOI of 100). Scale bars represent 25 µm. **c**, Schematic representation of the pCASP-HilA secretion system plasmid. The translation of the SptP specific chaperone SicP (in orange) and the fusion protein from (a or b) are coupled on a single mRNA and regulated by the *Salmonella* pSicA promoter. The main regulator of SPI-1 genes HilA is under the regulation of an arabinose inducible promoter (pBAD). Black boxes represent transcriptional termination and purple boxes with arrows represent promoters. Cm^{R}: chloramphenicol resistance, ColEl: plasmid origin of replication, Arac: Arabinose operon regulatory protein. d, Anti-FLAG tag Western blot of bacterial pellets and supernatants (SN) after 4-hour induction of *Salmonella* ASB2519 with or without arabinose in the last 2 hours, and HeLa cells infected with those *Salmonella* ASB2519 for 1 hour at a MOI of 100 in the presence or absence of arabinose. Anti-needle complex (T3SS) and anti-Actin blots serve as control for the presence of *Salmonella* and eukaryotic cells, respectively. The expected size of the SptP120-DARPin fusion protein is about 32 kDa. e, Flow cytometry analysis of 3 different FLAG-tagged SptP120-DARPins (E3_5 Control, α-GFP and α-mCherry DARPins) transferred into HeLa and HCT116 cells following 1 hour infection at a MOI of 100. Uninfected cells and *Salmonella* ASB2519 with an empty pCASP-HilA vector (ASB2519) served as negative controls. Experiments were repeated in three biological replicates showing comparable results. f, Quantitative anti-FLAG tag Western blot of HeLa and HCT116 cells from the same experiment as in (e). Numbers below the blots show quantification of the respective transferred SptP120-DARPin fusion proteins, based on 1.3 and 2.5 ng of a purified 3×FLAG protein (pure protein - RuvC) loaded on the same blot, and calculated with a measured single cell volume of 4.2 pL (HeLa) and 2.8 pL (HCT116), respectively. Note that the SptP mRNA sequence encoding the first nine amino acids is not translated because of the mRNA structure⁵².

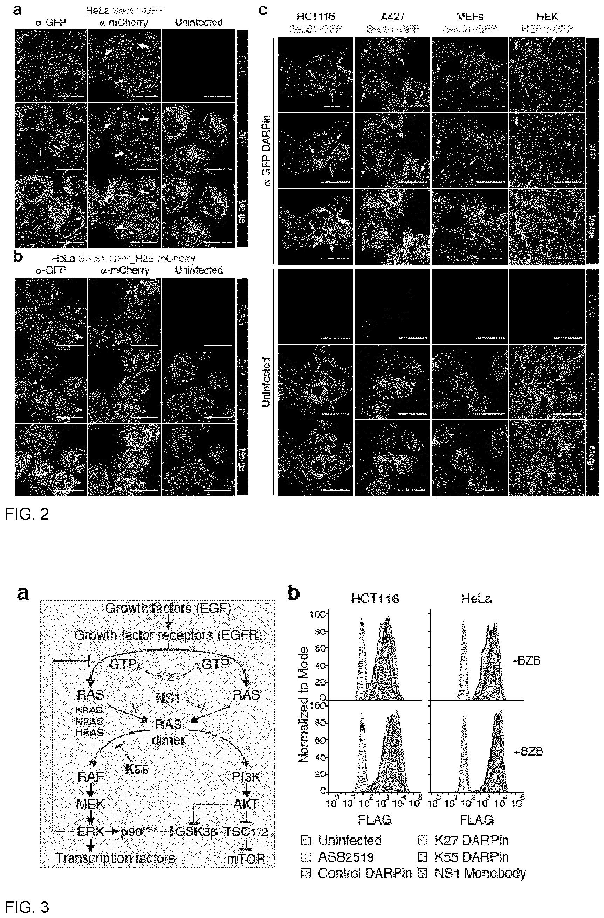

Figure 2 shows the delivery of functional DARPins into multiple cell types by Salmonella ASB2519. a,b, Representative anti-FLAG-immunostaining images (magenta, upper panels) of α-GFP and α-mCherry FLAG-tagged SptP120-DARPins transferred into HeLa cells (1 hour infection at a MOI of 100). Experiments were performed in HeLa cells expressing (a) Sec61-GFP or (b) both Sec61-GFP and H2B-mCherry. GFP (green) and mCherry (red) expression and merged images are also shown. c, Representative anti-FLAG-immunostainings of α-GFP FLAG-tagged SptP120-DARPin transferred into the indicated human and mouse cell types expressing Sec61-GFP and HER2-GFP. Experiments were repeated in three biological replicates showing comparable results. Uninfected samples served as negative controls. Scale bars represent 25 µm. Blue and orange arrows indicate colocalization in the cytoplasm and in nuclei, respectively. White arrows show that anti-FLAG does not co-localize with GFP using the α-mCherry DARPin.

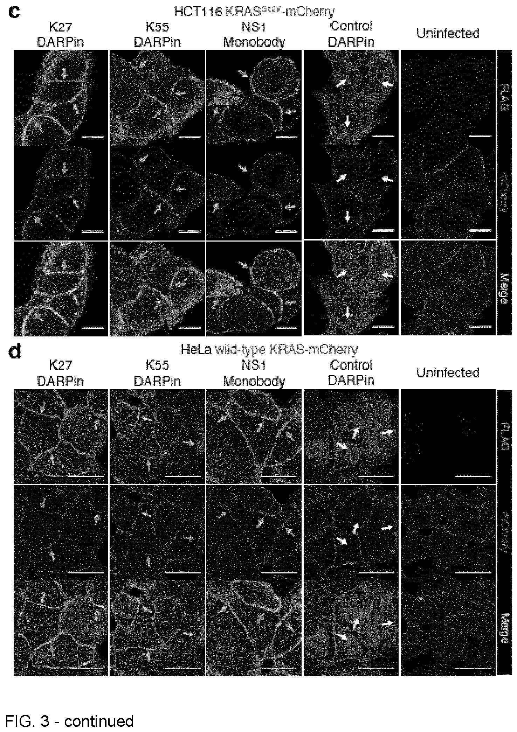

Figure 3 shows that the synthetic RAS inhibitory proteins are efficiently delivered into cells and co-localize with overexpressed mutated or wild-type KRAS proteins. a, Simplified overview of the DARPins (K27 and K55) and monobody (NS1) used to block RAS activation. Black arrows indicate activation and red lines show inhibition. b, Flow cytometry analysis of the FLAG-tagged SptP120-control DARPin and SptP120-anti-RAS inhibitors transferred into HCT116 and HeLa cells (1 hour infection, MOI of 100), in the absence (upper histograms) or presence (lower histograms) of the proteasome inhibitor bortezomib (BZB; 50 nM). Uninfected cells and cells infected with *Salmonella* ASB2519 with an empty pCASP-HilA vector (ASB2519) served as negative controls. c,d, Representative anti-FLAG-immunostainings (green) of FLAG-tagged SptP120-anti-RAS inhibitors transferred into HCT116 (c) and HeLa (d) cells expressing G12V- or wild-type-KRAS-mCherry. All samples were treated with BZB followed by 10 minutes incubation in fresh medium without (c) or with (d) EGF (20ng/ml) stimulation. Experiments were repeated in two biological replicates showing comparable results. Uninfected and SptP120-control DARPin served as negative controls. Scale bars are (c) 10 µm or (d) 25 µm. Blue arrows indicate colocalization. White arrows show that anti-FLAG does not co-localize with mCherry using the control DARPin.

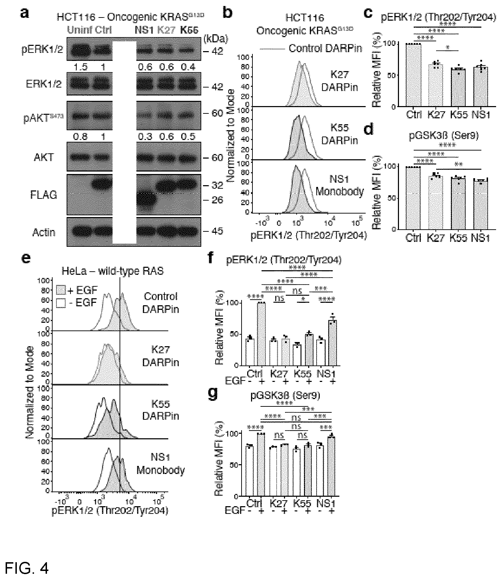

Figure 4 shows that T3SS-delivered RAS inhibitors downregulate RAS signaling. a, Western blot analysis of uninfected HCT116 cells (Uninf) and HCT116 cells infected with Salmonella ASB2519 (1 hour, MOI of 100) delivering a FLAG-tagged SptP120-control (Ctrl) DARPin and the SptP120-anti-RAS inhibitory DARPins K27 and K55 or the RAS blocking monobody NS1. Anti-FLAG detection served as the transfer control and total anti-ERK1/2, AKT and Actin as loading controls. Numbers below the pERK1/2 and pAKT blots detected with anti-pERK1/2 (Thr202/Tyr204) and anti-pAKT (Ser473) antibodies represent the quantified fraction relative to the SptP120-control DARPin (Ctrl), normalized to total ERK1/2 and AKT, respectively. b, Flow cytometric measurements of ERK1/2 phosphorylation in FLAG-positive HCT116 cells upon delivery of the indicated SptP120-anti-RAS binders. Data were analysed 10 minutes post-infection in the presence of bortezomib (BZB; 50 nM). Pink lines (-) indicate baseline ERK1/2 phosphorylation levels upon delivery of the SptP120-control DARPin. c,d, Relative median fluorescence intensities (MFI) of ERK1/2 (c) and GSK3□ (d) phosphorylation compared to the SptP120-control DARPin (Ctrl) treated cells and using the same experimental setup as in (b). e, Flow cytometric analysis of ERK1/2 phosphorylation in FLAG-positive HeLa cells upon delivery of the indicated SptP120-anti-RAS binders and a control DARPin. Data were analysed 10 minutes post-infection in the presence of bortezomib (BZB) without (empty) or with EGF (20 ng/ml) stimulation (filled). f,g, Relative median fluorescence intensities (MFI) of ERK1/2 (f) and GSK3□ (g) phosphorylation compared to the SptP120-control DARPin (Ctrl) treated cells with EGF and using the same experimental setup as in (e). Data represent the mean ± SEM of six (c,d) or three (f,g) biological replicates. Individual data points are shown. Statistical analysis was performed using a one-way ANOVA with Tukey's multiple comparisons test (****, P <0.0001; ***, P <0.001; **, P <0.01; *, P <0.05; ns, not significant; only relevant comparisons are indicated for (f) and (g)).

### EXAMPLES

The present invention is further illustrated by the following examples, which in no way should be construed as limiting. The entire contents of all of the references (including literature references, issued patents, published patent applications, and co pending patent applications) cited throughout this application are hereby expressly incorporated by reference.

### 1. Material & Methods

The material and methods applied in the studies of this invention are listed in the following, where necessary with reference to prior art references. For more details regarding the methods, the disclosure of the cited references should be considered.

### 1.1 Bacterial strains

The *Salmonella* strain SB2519^{ref.33} was transformed with the lambda red plasmid⁵⁴ and subsequently used to generate mutants using transformation with polymerase chain reaction (PCR) fragments amplified on the plasmid pKD3^{ref.55} with primers ACp255 + ACp256 for SopA (another known pathogenic T3SS-1 effector still present in SB2519) and primers ACp258 + ACp259 for the T3SS-2 indispensable protein SsaK. To generate the *Salmonella* strain ASB2519, mutations were subsequently transferred in a recipient *Salmonella* SB2519 strain by P22 phage transduction as described⁵⁶ and the resistance cassette flanked by FRT sites was flipped via transformation with the heat-sensitive pCP20 plasmid⁵⁷.

### 1.2 Gibson assembly

Primers for Gibson assembly were designed to have a length of 44 nucleotides, with 22 nucleotides for amplifying the sequence of interest and 22 nucleotides as annealing overhangs. Gradient-temperature PCR was performed with in-house Phusion polymerase at annealing temperatures calculated for the respective primers. PCR products were run on a 1% agarose gel in TAE buffer and purified with QIAquick Gel Extraction Kit (#28704, Qiagen). Equimolar amounts of vector and insert(s) were added to 10 µL of in-house Gibson assembly master mix in a total volume of 20 µL. The reaction was carried out for 1 hour at 50°C and incubated for 5 minutes on ice prior to transformation of chemically competent E. *coli* DH5□□produced in house. Plasmid DNA was prepared from positive clones and all constructs were confirmed by Sanger sequencing.

### 1.3 pCASP-HilA secretion plasmids and growth conditions

Secretion pCASP-HilA plasmids were obtained by Gibson assembly. The vector pCAS^{ref.30} was linearized by restriction digest of its single AvrII (NEB) site, and a fragment coding for an arabinose-inducible HilA construct was amplified by PCR with primers ACp100 + ACp101 from pSB667^{ref.39}. Subsequently, the pCASP-HilA vector was either amplified with the long SptP167 (ACp133 + ACp134) or the shortened SptP120 (ACp133 + ACp147) secretion tags. The inserts with the corresponding 22-nucleotide overhangs were ordered as gBlock fragments (Integrated DNA Technologies) and cloned with Gibson assembly. Strains carrying the pCASP-HilA plasmid were grown in LB medium (Sigma) containing 25 µg/mL chloramphenicol (Sigma) at 37°C with aeration.

### 1.4 Electrocompetent Salmonella

*S. typhimurium* were grown until reaching an optical density of 0.5 measured at a wavelength of 600 nm (OD600). After incubation on ice for 30 minutes, two washes with ultra-high pure water and two washes with 1x PBS (Gibco) containing 10% glycerol (AppliChem) were performed at 4°C. The electrocompetent *S. typhimurium* were resuspended in 1x PBS supplemented with 10% glycerol, aliquoted and immediately snap-frozen in liquid nitrogen and stored at -80°C. Electrocompetent *S. typhimurium* were electroporated with 1 µL of plasmid DNA in a pre-chilled electroporation cuvette (#1652086, BioRad) at 1,800 V with an electroporator (220V 940000017, Eppendorf). Super optimal broth with catabolite repression (SOC) medium (ThermoFisher) was added just after electroporation and *S. typhimurium* were grown for 1 hour at 37°C before plating on agar plates containing the respective antibiotic.

### 1.5 Salmonella secretion and infection assays

*Salmonella* were grown (directly from stable glycerol stocks) overnight at 37°C in LB with chloramphenicol at 220 rpm in a shaking incubator. The next morning, cultures were diluted 1 to 10 (1/10) in 5 mL of LB containing chloramphenicol and supplemented with 0.3 M NaCl to induce *Salmonella* pathogenicity island-1 (SPI-1) gene expression. After a 2 hours growth period, arabinose was added to a final concentration of 0.012% (weight/volume) to induce the expression of HilA and subsequent secretion of recombinant proteins fused to SptP120 or SptP167 in the following 2 hours. For secretion assays, cultures of *Salmonella* ASB2519 were grown for a total of 4 hours and then directly added to eukaryotic cells at a multiplicity of infection (MOI) of 100. Infections were carried out for 1 hour in the respective eukaryotic cell culture medium supplemented with 25 µg/mL chloramphenicol and 0.012% (w/v) arabinose. After a 1-hour infection, *Salmonella* ASB2519 were removed and eukaryotic cells washed once with 1x PBS and further incubated without *Salmonella* ASB2519 for 10 minutes either without or with 20 ng/mL EGF or for 3 hours with 200 µg/mL gentamycin and during the last 10 minutes without or with 20 ng/mL EGF. When indicated, the proteasome inhibitor bortezomib (BZB) (Abcam) was applied at 50 nM for 30 minutes prior to infection and sustained throughout the entire experiment. The cytotoxicity of our *Salmonella* strain ASB2519 delivering SptP120 fused to DARPins was analyzed via lactate dehydrogenase cytotoxicity assay LDH. LDH release from infected and uninfected eukaryotic cells was measured with the CytoTox 96 Non-Radioactive Cytotoxicity Assay (G1780, Promega) following the manufacturer's instructions. The relative amount of LDH release was calculated as follows: percentage released LDH (sample) = (sample - medium background) / (maximum LDH - medium background) ×100.

### 1.6 Protein purification

As control for the Western blot analysis, 3×FLAG-RuvC protein was expressed in the *E. coli* strain BL21(DE3) harboring the plasmid pET-52b(+) carrying the coding sequence corresponding to RuvC derived from *S. typhimurium.* Cells were grown in LB broth supplemented with 100 µg/ml ampicillin at 37°C to an OD600 of 0.6. Expression of RuvC was induced by the addition of 1 mM IPTG and cultures were further incubated at 37°C for 3 hours. Subsequently, cells were collected by centrifugation, washed in wash buffer [20 mM Tris-HCI (pH 8), 20 mM NaCl and 1 mM EDTA], resuspended in lysis buffer [100 mM Tris-HCI, pH 8, 500 mM NaCl and 10% glycerol] and stored at -80°C. When required, cells were lysed by sonication and resulting suspension was centrifuged at 37,000 rpm for 1 hour at 4°C. Cell-free lysate was applied onto a HisTrap HP column equilibrated with buffer 1 [100 mM Tris-HCI (pH 8), 10% glycerol and 500 mM NaCl] and bound proteins were eluted with a linear gradient of 10 mM → 500 mM imidazol. Peak fractions were pooled and dialyzed against buffer 1. The protein pool was loaded onto a Superdex 200 column equilibrated with buffer 2 (100 mM Tris pH 8, 500 mM NaCl, 1 mM EDTA, 0.5 mM DTT, 15% glycerol). The peak containing RuvC protein eluted at 15 ml, was collected and stored at 80°C. The purity of RuvC was >90%, as judged by SDS-PAGE followed by staining with Coomassie blue (data not shown).

### 1.7 Western blots

*Salmonella* from a 5 mL culture (also used in the secretion assays) were pelleted at 4°C and 6,000 g for 15 minutes. The bacterial culture supernatants were harvested and 0.2 µm filtered (supernatant secretion samples) while the *Salmonella* pellets were resuspended in 5 mL of 1x PBS and mixed in a 1/1 ratio with 87% (volume/volume) glycerol (expression samples). Secretion and expression samples were analyzed without a concentration step. For Western blot analysis, eukaryotic cells were infected in 6-well plates. At the end of the experiment, cells were washed once with 1x PBS; to harvest cells Accutase (A1110501, ThermoFisher) was added for 10 minutes at 37°C. After centrifugation at 4°C and 2,000 *g* for 2 minutes, cell pellets were immediately frozen in liquid nitrogen and stored at -80°C. Cell pellets were thawed on ice and resuspended in 1x PBS with 0.002% digitonin (Sigma) and supplemented with protease inhibitor cocktail (cOmplete, Roche). Resuspended pellets were kept on ice for 5 minutes and centrifuged at 4°C and 16,000 *g* for 25 minutes. Since digitonin does not lyse bacterial membranes, supernatant containing only eukaryotic cells content was harvested and the total protein content determined by a Bradford protein assay (#5000001, BioRad). Samples were separated on a 4-12% gradient NuPAGE gel (NP0335BOX, ThermoFisher) and blotted on polyvinylidene difluoride (PVDF) membrane by wet transfer. PVDF membranes were probed with: mouse monoclonal M2 anti-FLAG (1/3,000 dilution, F1804, Sigma), mouse anti-actin (1/1,000 dilution, A5316, Sigma), rabbit anti-phospho-ERK1/2 (Thr202/Tyr204) (1/1,000 dilution, #9101, Cell Signaling), rabbit anti-ERK1/2 (1/1,000 dilution, #9102, Cell Signaling), rabbit anti-phospho-AKT (Ser473) (1/1,000 dilution, #9271, Cell Signaling), rabbit anti-AKT (1/1,000 dilution, #4685, Cell Signaling), or rabbit anti-T3SS (1/3,000 dilution, in house generated) antibodies in milk or phosphoblocker (AKR-103-S, Cell Biolabs) diluted in washing buffer (1x TBS-0.1% Tween-20) overnight at 4°C. Detection was performed with either secondary anti-mouse IgG (1/4,000 dilution, W4021, Promega) or anti-rabbit IgG (1/4,000 dilution, NA9340, Sigma) antibodies conjugated to horseradish peroxidase, and visualized using enhanced chemiluminescence (ECL Plus, #32132, ThermoFisher).

### 1.8 Lentiviral vector production and infection

Lenti-mCherry-G12V-KRAS-IRES-Blast and Lenti-mCherry-WT-KRAS-IRES-Blast were obtained by Gibson assembly. The vector was amplified by PCR with primers ACp326 + ACp327 from the Lenti-AcGFP-Sec61-IRES-Blast plasmid, mCherry with primers ACp328 + ACp329 from a plasmid containing mCherry, and KRAS with primers ACp330 + ACp331 from wild-type or G12V-KRAS containing plasmids (gift from Boehringer Ingelheim). Lentiviral vectors were produced in 6-well plates as previously described⁵⁸ by transfection of Lenti-X 293T cells with 4 µg of plasmid vector (Lenti-AcGFP-Sec61-IRES-Blast, Lenti-mCherry-G12V-KRAS-IRES-Blast or Lenti-mCherry-WT-KRAS-IRES-Blast), packaging plasmid pCMVR8.74 and vesicular stomatitis G producing plasmid at a ratio of 4:2:1. Culture medium was collected 24 hours post-transfection, 0.2 µm filtered and directly used to infect recipient cells for 24 hours. This procedure was repeated a second time 48 hours post-transfection. Transduced cells were selected for blasticidin (ThermoFisher) resistance resulting in a mixed population of integration-positive cells expressing various level of either GFP tagged Sec61 subunit beta or mCherry tagged oncogenic (G12V) or wild-type KRAS.

### 1.9 Cell culture

All cell lines used in this study were grown in high glucose DMEM (Gibco) supplemented with 10% fetal bovine serum (FBS) (Gibco), non-essential amino acids, 2 mM L-glutamine (Sigma) and 25 mM HEPES (Gibco).

### 1.10 Immunofluorescence

Cells were seeded on pre-treated (70% ethanol, 0.1% gelatin 30 minutes) coverslips (#72196-12, EMS) in 24-well plates the day before the infection experiment. At the end of the infection experiment, cells were washed once with 1x PBS and subsequently fixed with 4% paraformaldehyde (PFA) (#28908, ThermoFisher) for 10 minutes at room temperature (RT) and permeabilized and blocked for 1 hour at RT in blocking buffer (1x PBS supplemented with 5% FBS, 2% bovine serum albumin (BSA), 1% glycine and 0.2% Triton X-100). The specimens were stained with primary mouse monoclonal M2 anti-FLAG antibodies (1/1,000 dilution, F1804, Sigma) overnight at 4°C in a humid chamber, washed three times for 15 minutes at RT with washing buffer (1x PBS supplemented with 0.2% Triton X-100, 1% glycine) and stained with secondary Alexa 647-labelled anti-mouse IgG (1/500 dilution, ab150115, Abcam). Specimens were again washed three times for 15 minutes at RT and nuclei were counterstained with DAPI (1/2,000 dilution, D3571, ThermoFisher). Finally, specimens were mounted onto cover glasses (SuperFrost Ultra Plus, ThermoFisher) with fluorescence mounting medium (S3023, Dako) and confocal images were taken using a Carl Zeiss LSM880 microscope with Airyscan.

### 1.11 Flow cytometry

Cells were seeded in 96-well plates the day before the infection. Following the infection experiments, cells were washed once with 1x PBS and Accutase was added for 10 minutes at 37°C for harvesting. Cells were subsequently stained with the fixable viability dye eFluor^{™} 780 (1/1,000 dilution, 65-0865, ThermoFisher) for 15 minutes on ice and fixed with 4% PFA for 10 minutes at RT. Sample were permeabilized in blocking buffer for 30 minutes at RT and stained with primary the mouse monoclonal M2 anti-FLAG antibody (1/1,000 dilution, F1804, Sigma) followed by directly PE-labelled secondary anti-mouse IgG F(ab')2 antibody (1/1,000 dilution, 12-4010-87, ThermoFisher) in blocking buffer, each for 20 minutes at RT. For phosphorylation analyses, samples were fixed with BD Cytofix (#554655, BDbiosciences) for 15 minutes at 37°C and permeabilized in Perm/Wash buffer (#557885, BDbiosciences) for 20 minutes at RT. Samples were stained with directly labelled FITC-anti-FLAG M2 (1/50 dilution, F4049, Sigma), PE-anti-phospho-ERK (Thr202/Tyr204) (1/100 dilution, #369506, biolegend) or Pacific Blue-anti-phospho-GSK3□ (Ser9) (1/100 dilution, #14310, Cell signaling) for 1.5 hours at RT or overnight at 4°C in a humid chamber. Samples were analyzed with a FACS LSR Fortessa (BDbiosciences). To compare experiments performed on different days, we set the median fluorescence intensity (MFI) of cells receiving the Control DARPin (with EGF induction of the cells when needed) to 100% per condition and phosphorylation marker and subsequently calculated the relative percentage MFI under the different experimental conditions.

### 1.12 Statistics

If not otherwise stated, all values in this study are given as means ± SEM. GraphPad Prism software was used to create bar graphs and perform statistical analyses. Data were analyzed by using a one-way ANOVA with Tukey's multiple comparisons test, as indicated. P < 0.05 was considered as statistically significant.

### 2. Results

The results that were obtained from the experiments conducted in this study are described in more detail below.

### 2.1 Engineering of a bacterial protein system for delivery into eukaryotic cells

To induce secretion of a synthetic protein from *Salmonella,* we took advantage of the previously developed pCASP plasmid system³⁰. In this system, the expression of recombinant proteins is under the control of the pSicA promoter and thus co-regulated with *Salmonella* pathogenicity island-1 (SPI-1) gene expression. Recombinant proteins were tagged with a 3×FLAG tag at their C-termini and at their N-termini with the first 167 amino acids of the natural *Salmonella* effector protein SptP (SptP167) for secretion (Fig. 1a). SptP167 contains both a secretion signal and a chaperone binding domain (CBD)³¹ - two domains that have previously been shown to be sufficient and necessary for secretion by T3SS³². Moreover, we mutated another known pathogenic T3SS-1 effector (SopA) and the T3SS-2 indispensable protein SsaK in the "effectorless" *S. typhimurium* strain SB2519 (generously provided by the Galan laboratory³³) to generate a novel non-toxic strain called ASB2519, carrying a disrupted T3SS-2 to block its capacity to replicate intracellularly in case of phagocytosis³⁴.

Human HeLa cells (human cervix adenocarcinoma cells) and HCT116 cells (human colorectal carcinoma cells) were infected with this non-toxic ASB2519 strain of *S. typhimurium,* carrying the pCASP-SptP167-3G124-HiIA plasmid to express and deliver the anti-GFP DARPin 3G124³⁵ fused to the SptP167 secretion tag. We observed effective transfer of the α-GFP DARPin; however, the transferred binders predominantly localized at the plasma membrane of both tested eukaryotic cell types (Fig. 1a). Such membrane localization was also seen using a different DARPin (called E3_5, which has no defined binding specificity³⁶, data not shown), suggesting that the addition of SptP167 results in mis-localization of the delivered affinity reagents. For this reason, and based on the crystal structure of SptP-CBD bound to a dimer of its specific chaperone SicP³¹, we therefore designed a shortened SptP secretion tag termed SptP120 (terminated at threonine 120encompassing the first three, but excluding the forth, interaction of SptP-CBD with the SicP dimer³¹). Importantly, in contrast to the membrane mis-localization found for full-length SptP167-fused DARPins, this SptP120 secretion tag resulted in efficient intracellular delivery as well as the predicted cytoplasmic and nuclear localization (Fig. 1b).

To boost T3SS-1 expression in *Salmonella* and thereby permit delivery of large amounts of DARPins, we inserted the known key regulator of SPI-1 genes, HilA³⁷⁻³⁹, into the pCASP vector under an arabinose inducible promoter (pBAD). Using this novel pCASP-HilA vector (Fig. 1c) and following the induction with arabinose, a marked increase in both DARPin secretion into the supernatant (SN) from *Salmonella* cultures and cytoplasmic transfer of DARPins into HeLa cells could be detected (Fig. 1d). Using flow cytometry, we further confirmed the efficient transfer of the α-GFP DARPin into both HeLa as well as HCT116 cells. Importantly, the modified system reproducibly delivered DARPins into > 95% of the cells (Fig. 1e). These data were confirmed using two additional DARPin binders (Fig. 1e), the control DARPin E3_5 as well as an α-mCherry binder (3m160)³⁵. Intracellular delivery of DARPins was further demonstrated by quantitative Western blotting, indicating that around 1.9 ng and 1.7 ng of each DARPin could be delivered into 75,000 HeLa and 150,000 HCT116 cells, respectively, after a 1 hour infection at a multiplicity of infection (MOI) of 100 (Fig. 1f). This equals a concentration of 188 nM in HeLa cells and 126 nM in HCT116 cells, with measured single cell volumes of 4.2 pL and 2.8 pL, repectively. Of note, intracellular delivery of the SptP120 DARPins did not show any apparent cytotoxicity in HeLa and HCT116 cells. Thus, this novel *S. typhimurium* T3SS delivery system enables highly efficient cytosolic transfer of DARPins.

### 2.2 Cytoplasmic delivery of functional DARPins

Effector proteins need to unfold to pass through the narrow needle complex channel of T3SS and, once transferred, the proteins need to refold in the cytoplasm of the eukaryotic cell to be functional⁴⁰. Our data so far have demonstrated efficient intracellular delivery of DARPins, indicating that they are properly unfolded for secretion, despite the known high stability of these proteins^{36,41}. Once inside the eukaryotic cells, DARPins need to refold to be functional and bind their targets. To validate regain of function after transfer, we delivered the previously used α-GFP DARPin into HeLa cells constitutively overexpressing either the endoplasmic reticulum (ER) transport protein Sec61 subunit beta fused with GFP on their cytoplasmic N-terminus (Sec61-GFP) or the nuclear histone protein H2B fused to GFP (H2B-GFP). When we probed for the transferred α-GFP DARPin, we indeed observed its expected co-localization (detected by its FLAG-tag) with GFP at the cytoplasmic membrane of the ER for Sec61-GFP or in the nucleus for H2B-GFP, respectively (Fig. 2a and). As a control, the α-mCherry DARPin 3m160³⁵ did not co-localize with GFP (Fig. 2a). To ensure that the system generally can transfer functional DARPins, we next delivered them into HeLa cells overexpressing the cytoskeleton protein alpha-Tubulin-mCherry. The α-mCherry DARPin, but not the α-GFP binder co-localized with the mCherry-labelled cytoskeleton protein alpha-Tubulin. Finally, we delivered both DARPins into HeLa cells co-expressing both Sec61-GFP and H2B-mCherry. The α-GFP DARPin co-localized with GFP on the ER membranes while the α-mCherry DARPin did bind to the mCherry-fusion protein in the nucleus (Fig. 2b), confirming specificity of the transferred DARPins.

To test whether our new system enables the delivery of functional DAPRins in multiple cell types, we generated HCT116, A427 (human lung carcinoma cells), and immortalized mouse embryonic fibroblasts (MEFs) that constitutively express Sec61-GFP. Moreover, we tested HER2-GFP expressing human HEK293 embryonic kidney cells, where GFP is fused to the C-terminus and is thus located on the cytosolic side of the plasma membrane. In all five different cell lines from two different species (mouse and human), we observed delivery of α-GFP DARPin and its correct co-localization with the overexpressed target-GFP-fusion (Fig. 2c). Furthermore, in HEK293 cells co-localization of the α-GFP DARPin with the membrane protein HER2 could be detected (Fig. 2c). Of note, although DARPins generally are biophysically stable proteins, we detected reduced levels of the bacteria-delivered proteins within all the tested cells at ∼ 3 hours post-infection; addition of the proteasome inhibitor bortezomib (BZB)⁴² rescued the levels of the intracellular delivered fusion-proteins. Importantly, BZB treatment had no apparent effect on the specific co-localization of DARPins to their respective targets. These data show DARPins can be delivered into multiple cell types and that the transferred DARPins are functional and co-localize with their specific target proteins.

### 2.3 Bacterial delivery of recombinant binders to inhibit RAS signaling

Having performed proof-of-concept experiment with the α-GFP and α-mCherry DARPins, we next aimed to use our system to deliver synthetic proteins to inhibit RAS activity. RAS is a key pathway of multiple growth factor signaling cascades and one of the most frequently mutated genes in human cancer^{43,44}. With the exception of the *KRAS^{G12C}* mutation, for which a covalent inhibitor has been described, all other oncogenic KRAS mutants still remain undruggable⁴⁵. Two DARPins (K27 and K55)²⁴ and a monobody (NS1)²⁵ have been recently developed to block RAS and overexpression of these inhibitory proteins (using > 24 hours transfections and stable integrations) has been reported to downregulate activation of the RAS pathway in cell lines harboring *RAS* mutations, yet by different modes of action: K27 inhibits RAS-GTP nucleotide exchange and K55 out-competes the downstream effector RAF while NS1 prevents RAS dimerization (Fig. 3a).

To test whether our bacterially-delivered affinity reagents can inhibit RAS signaling, HCT116 cells were infected for 1 hour with the *Salmonella* strain ASB2519 delivering either K27, K55, NS1 or the control DARPin E3_5. We observed efficient delivery of all DARPins and also the monobody into HCT116 as well as HeLa cells (Fig. 3b). Proteasome inhibition again resulted in higher levels of the delivered proteins (Fig. 3b). To confirm that the bacterially-delivered DARPins and the NS1 monobody were binding to their targets, we delivered them in HCT116 cells that were engineered to stably express an oncogenic KRAS (G12V) fused to mCherry at its N-terminus as well as in HeLa cells expressing mCherry tagged wild-type KRAS. Both DARPins and the NS1 monobody co-localized with overexpressed mCherry-tagged oncogenic KRAS (Fig. 3c) as well as with activated wild-type KRAS (Fig. 3d). Thus, all synthetic RAS inhibitory proteins we tested can be efficiently transferred and faithfully co-localize to their specific target.

HCT116 cells harbor a G13D activating mutation of *KRAS* (KRAS^{G13D}) resulting in a constitutively activated KRAS pathway⁴⁶. Delivery of the two DARPins and the NS1 monobody into HCT116 tumor cells caused a markedly reduced phosphorylation of ERK1/2 (pERK1/2 Thr202/Tyr204) and AKT (pAKT Ser473) (Fig. 4a). Inhibition of the constitutively active RAS pathway in HCT116 cells was confirmed in multiple independent experiments by intracellular flow cytometry to detect active phosphorylated ERK1/2 at 10 minutes (Fig. 4b,c) and 3 hours post-infection. Moreover, we noted reduced phosphorylation of the ERK1/2 and AKT downstream mediator GSK3□□(pGSK3□ Ser9) (see Fig. 3a) by flow cytometry following intracellular delivery of both DARPins and the monobody (Fig. 4d). Of note, since the delivery of the control DARPin also resulted in a minor downregulation of ERK1/2 activation, we always used this control setting for the flow cytometry experiments.

To examine the inhibitory effects of these proteins on EGF-mediated induction of wild-type RAS, we used HeLa cells which carry wild-type RAS alleles. Bacterially-delivered anti-RAS DARPins again markedly abrogated EGFR-induced ERK1/2 and GSK3□ phosphorylation, as determined by intracellular immunostaining (Fig. 4e-g). Importantly, whereas the NS1 monobody inhibited RAS activation to a similar level as both DARPins in HCT116 cells carrying an activating KRAS mutation, the NS1 monobody showed a significantly reduced capacity to block EGFR-induced activation of wild-type RAS (Fig. 4). These data show that our system allows for the efficient transfer of DARPins as well as monobodies into human cancer cells. Importantly, such bacterially-delivered synthetic binding proteins can significantly block the activation of oncogenic KRAS as well as growth factor-induced wild-type RAS.

### LITERATURE

1. Vivès, E., Brodin, P. & Lebleu, B. A truncated HIV-1 Tat protein basic domain rapidly translocates through the plasma membrane and accumulates in the cell nucleus. J. Biol. Chem. 272, 16010-7 (1997).
2. Derossi, D., Joliot, A. H., Chassaing, G. & Prochiantz, A. The Third Helix of the Antennapedia Homeodornain Translocates through Biological Membranes. J. Biol. Chem. 296, 10444-10450 (1994).
3. Deprey, K., Becker, L., Kritzer, J. & Plückthun, A. Trapped! A Critical Evaluation of Methods for Measuring Total Cellular Uptake versus Cytosolic Localization. Bioconjug. Chem. 30, 1006-1027 (2019).
4. Kamaly, N., Xiao, Z., Valencia, P. M., Radovic-Moreno, A. F. & Farokhzad, O. C. Targeted polymeric therapeutic nanoparticles: design, development and clinical translation. Chem. Soc. Rev. 41, 2971-3010 (2012).
5. Rabideau, A. E. & Pentelute, B. L. Delivery of Non-Native Cargo into Mammalian Cells Using Anthrax Lethal Toxin. ACS Chem. Biol. 11, 1490-1501 (2016).
6. Beilhartz, G. L., Sugiman-Marangos, S. N. & Melnyk, R. A. Repurposing bacterial toxins for intracellular delivery of therapeutic proteins. Biochem. Pharmacol. 142, 13-20 (2017).
7. Verdurmen, W. P. R., Mazlami, M. & Plückthun, A. A quantitative comparison of cytosolic delivery via different protein uptake systems. Sci. Rep. 7, 1-13 (2017).
8. Galán, J. E. & Waksman, G. Protein-Injection Machines in Bacteria. Cell 172, 1306-1318 (2018).
9. Galán, J. E., Lara-Tejero, M., Marlovits, T. C. & Wagner, S. Bacterial type III secretion systems: specialized nanomachines for protein delivery into target cells. Annu. Rev. Microbiol. 68, 415-38 (2014).
10. Plückthun, A. Designed Ankyrin Repeat Proteins (DARPins): Binding Proteins for Research, Diagnostics, and Therapy. Annu. Rev. Pharmacol. Toxicol. 55, 489-511 (2015).
11. Muyldermans, S. Nanobodies: natural single-domain antibodies. Annu. Rev. Biochem. 82, 775-97 (2013).
12. Sha, F., Salzman, G., Gupta, A. & Koide, S. Monobodies and other synthetic binding proteins for expanding protein science. Protein Sci. 26, 910-924 (2017).
13. Herce, H. D. et al. Cell-permeable nanobodies for targeted immunolabelling and antigen manipulation in living cells. Nat. Chem. 1-10 (2017). doi:10.1038/nchem.2811
14. Tabtimmai, L. et al. Cell-penetrable nanobodies (transbodies) that inhibit the tyrosine kinase activity of EGFR leading to the impediment of human lung adenocarcinoma cell motility and survival. J. Cell. Biochem. 1-11 (2019). doi:10.1002/jcb.29111
15. Röder, R. et al. Intracellular Delivery of Nanobodies for Imaging of Target Proteins in Live Cells. Pharm. Res. 34, 161-174 (2017).
16. Liao, X., Rabideau, A. E. & Pentelute, B. L. Delivery of Antibody Mimics into Mammalian Cells via Anthrax Toxin Protective Antigen. Chembiochem 2458-2466 (2014). doi:10.1002/cbic.201402290
17. Schmit, N. E., Neopane, K. & Hantschel, O. Targeted Protein Degradation through Cytosolic Delivery of Monobody Binders Using Bacterial Toxins. ACS Chem. Biol. 14, 916-924 (2019).
18. Verdurmen, W. P. R., Luginbühl, M., Honegger, A. & Plückthun, A. Efficient cell-specific uptake of binding proteins into the cytoplasm through engineered modular transport systems. J. Control. Release 200, 13-22 (2015).
19. Ittig, S. J. et al. A bacterial type III secretion-based protein delivery tool for broad applications in cell biology. J. Cell Biol. 211, 913-931 (2015).
20. Blanco-Toribio, A., Muyldermans, S., Frankel, G. & Fernández, L. Á. Direct injection of functional single-domain antibodies from E. coli into human cells. PLoS One 5, e15227 (2010).
21. Bai, F., Li, Z., Umezawa, A., Terada, N. & Jin, S. Bacterial type III secretion system as a protein delivery tool for a broad range of biomedical applications. Biotechnol. Adv. 36, 482-493 (2018).
22. Jin, Y. et al. Enhanced differentiation of human pluripotent stem cells into cardiomyocytes by bacteria-mediated transcription factors delivery. PLoS One 13, 1-14 (2018).
23. Jia, J. et al. Efficient Gene Editing in Pluripotent Stem Cells by Bacterial Injection of Transcription Activator-Like Effector Nuclease Proteins. Stem Cells Transl. Med. 4, 913-26 (2015).
24. Guillard, S. et al. Structural and functional characterization of a DARPin which inhibits Ras nucleotide exchange. Nat. Commun. 8, 16111 (2017).
25. Spencer-Smith, R. et al. Inhibition of RAS function through targeting an allosteric regulatory site. Nat. Chem. Biol. 13, 62-68 (2017).
26. Tanaka, T., Williams, R. L. & Rabbitts, T. H. Tumour prevention by a single antibody domain targeting the interaction of signal transduction proteins with RAS. EMBO J. 26, 3250-9 (2007).
27. Bery, N. et al. KRAS-specific inhibition using a DARPin binding to a site in the allosteric lobe. Nat. Commun. 10, 0-10 (2019).
28. Tamaskovic, R. et al. Intermolecular biparatopic trapping of ErbB2 prevents compensatory activation of PI3K/AKT via RAS-p110 crosstalk. Nat. Commun. 7, 11672 (2016).
29. Kummer, L. et al. Knowledge-based design of a biosensor to quantify localized ERK activation in living cells. Chem. Biol. 20, 847-856 (2013).
30. Widmaier, D. M. et al. Engineering the Salmonella type III secretion system to export spider silk monomers. Mol. Syst. Biol. 5, 309 (2009).
31. Stebbins, C. E. & Galán, J. E. Maintenance of an unfolded polypeptide by a cognate chaperone in bacterial type III secretion. Nature 414, 77-81 (2001).
32. Lee, S. H. & Galán, J. E. Salmonella type III secretion-associated chaperones confer secretion-pathway specificity. Mol. Microbiol. 51, 483-95 (2004).
33. Spano, S., Gao, X., Hannemann, S., Lara-Tejero, M. & Galan, J. E. A Bacterial Pathogen Targets a Host Rab-Family GTPase Defense Pathway with a GAP. Cell Host Microbe 19, 216-226 (2016).
34. Fàbrega, A. & Vila, J. Salmonella enterica serovar Typhimurium skills to succeed in the host: Virulence and regulation. Clin. Microbiol. Rev. 26, 308-341 (2013).
35. Brauchle, M. et al. Protein interference applications in cellular and developmental biology using DARPins that recognize GFP and mCherry. Biol. Open 3, 1252-1261 (2014).
36. Kohl, A. et al. Designed to be stable: Crystal structure of a consensus ankyrin repeat protein. Proc. Natl. Acad. Sci. 100, 1700-1705 (2003).
37. Bajaj, V., Lucas, R. L., Hwang, C. & Lee, C. A. Co-ordinate regulation of Salmonella typhimurium invasion genes by environmental and regulatory factors is mediated by control of hilA expression. Mol. Microbiol. 22, 703-714 (1996).
38. Bajaj, V., Hwang, C. & Lee, C. A. hilA is a novel ompR/toxR family member that activates the expression of Salmonella typhimurium invasion genes. Mol. Microbiol. 18, 715-727 (1995).
39. Eichelberg, K. & Galán, J. E. Differential regulation of Salmonella typhimurium type III secreted proteins by pathogenicity island 1 (SPI-1)-encoded transcriptional activators InvF and hilA. Infect. Immun. 67, 4099-105 (1999).
40. Radics, J., Königsmaier, L. & Marlovits, T. C. Structure of a pathogenic type 3 secretion system in action. Nat. Struct. Mol. Biol. 21, 82-7 (2014).
41. Wetzel, S. K., Settanni, G., Kenig, M., Binz, H. K. & Plückthun, A. Folding and Unfolding Mechanism of Highly Stable Full-Consensus Ankyrin Repeat Proteins. J. Mol. Biol. 376, 241-257 (2008).
42. Teicher, B. A. et al. The Proteasome Inhibitor PS-341 in Cancer Therapy The Proteasome Inhibitor PS-341 in Cancer Therapy. 5, 2638-2645 (1999).
43. Prior, I. a, Lewis, P. D. & Mattos, C. A comprehensive survey of Ras mutations in cancer. Cancer Res. 72, 2457-67 (2012).
44. Simanshu, D. K., Nissley, D. V. & McCormick, F. RAS Proteins and Their Regulators in Human Disease. Cell 170, 17-33 (2017).
45. McCormick, F. Progress in targeting RAS with small molecule drugs. Biochem. J. 476, 365-374 (2019).
46. Schroy, P. C. et al. Detection of p21ras mutations in colorectal adenomas and carcinomas by enzyme-linked immunosorbent assay. Cancer 76, 201-9 (1995).
47. Schilling, J., Schöppe, J. & Plückthun, A. From DARPins to LoopDARPins: Novel LoopDARPin design allows the selection of low picomolar binders in a single round of ribosome display. J. Mol. Biol. 426, 691-721 (2014).
48. Verdurmen, W. P. R., Luginbühl, M., Honegger, A. & Plückthun, A. Efficient cell-specific uptake of binding proteins into the cytoplasm through engineered modular transport systems. J. Control. Release 200, 13-22 (2015).
49. Niemann, G. S. et al. Discovery of novel secreted virulence factors from salmonella enterica serovar typhimurium by proteomic analysis of culture supernatants. Infect. Immun. 79, 33-43 (2011).
50. Kubori, T. & Galán, J. E. Temporal regulation of Salmonella virulence effector function by proteasome-dependent protein degradation. Cell 115, 333-42 (2003).
51. Schraidt, O. & Marlovits, T. C. Three-dimensional model of Salmonella's needle complex at subnanometer resolution. Science 331, 1192-5 (2011).
52. Button, J. E. & Galán, J. E. Regulation of chaperone/effector complex synthesis in a bacterial type III secretion system. Mol. Microbiol. 81, 1474-1483 (2011).
53. Hoiseth, S. K. & Stocker, B. A. Aromatic-dependent Salmonella typhimurium are non-virulent and effective as live vaccines. Nature 291, 238-9 (1981).
54. Kenan C Murphy and Kenneth G Campellone. Lambda Red-mediated recombinogenic engineering of\nenterohemorrhagic and enteropathogenic E. coli. Biomed Cent. Mol. Biol. 4, 12 (2003).
55. Datsenko, K. a & Wanner, B. L. One-step inactivation of chromosomal genes in Escherichia coli K-12 using PCR products. Proc. Natl. Acad. Sci. USA 97, 6640-6645 (2000).
56. Kaniga, K., Bossio, J. C. & Galán, J. E. The Salmonella typhimurium invasion genes invF and invG encode homologues of the AraC and PulD family of proteins. Mol. Microbiol. 13, 555-568 (1994).
57. Cherepanov, P. P. & Wackernagel, W. Gene disruption in Escherichia coli: TcR and KmR cassettes with the option of Flp-catalyzed excision of the antibiotic-resistance determinant. Gene 158, 9-14 (1995).
58. Barde, I., Salmon, P. & Trono, D. Production and titration of lentiviral vectors. Curr. Protoc. Neurosci. Chapter 4, Unit 4.21 (2010).
59. Alix, E., Mukherjee, S. & Roy, C. R. Subversion of membrane transport pathways by vacuolar pathogens. J. Cell Biol. 195, 943-952 (2011).
60. Steigemann, P. et al. Aurora B-Mediated Abscission Checkpoint Protects against Tetraploidization. Cell 136, 473-484 (2009).

## Claims

1. Fusion protein, comprising
(a) a protein translocation domain consisting of amino acids 1-165 of the *Salmonella* protein SptP or a fragment thereof; and
(b) a protein or protein domain for delivery into a eukaryotic cell.

2. Fusion protein of claim 1, wherein said protein translocation domain is derived from the protein SptP of *S*. *typhimurium.*

3. Fusion protein of claim 1 or 2, wherein said protein translocation domain consists of amino acids 1-165 of SEQ ID NO:1 or a fragment thereof.

4. Fusion protein of any of claims 1-3, wherein said fragment of the protein translocation domain comprises or consists of amino acids 1-100, amino acids 1-110, amino acids 1-120, amino acids 1-130, amino acids 1-140, or amino acids 1-150 of the sequence of SEQ ID NO:1 or a fragment of any of these.

5. Fusion protein of any of claims 1-4, wherein the fusion protein further comprises a linker between the protein translocation domain and the protein or protein domain that is to be delivered to the eukaryotic cell.

6. Fusion protein of any of claims 1-5, wherein the protein or protein domain that is to be delivered to the eukaryotic cell is a target-binding protein.

7. Fusion protein of any of claims 1-6 for use in medicine.

8. Pharmaceutical composition comprising a fusion protein of any of claims 1-6.

9. Polynucleotide encoding a fusion protein of any of claims 1-6.

10. Expression vector comprising the polynucleotide of claim 9.

11. Host cell comprising the polynucleotide of claim 9 or the expression vector of claim 10.

12. Method for preparing the fusion protein of any of claims 1-6, comprising culturing of a host cell of claim 10 under conditions which allow for the expression of the fusion protein.

13. Method of introducing a protein of interest, such as a target-binding protein or therapeutically active protein, into a eukaryotic cell, comprising contacting the eukaryotic cell with a fusion protein of any of claim 1-6.

14. Method of claim 13, wherein the fusion protein is introduced by means of a type III secretion system (T3SS).

15. Use of a protein translocation domain consisting of amino acids 1-165 of the *Salmonella* protein SptP or a fragment thereof for introducing a protein of interest, such as a therapeutically active protein, into a eukaryotic cell.


SEQUENCE LISTING

<110>  Universitätsklinikum Hamburg-Eppendorf

<120>  NOVEL PROTEIN TRANSLOCATION DOMAIN

<130>  P 109921

<160>  7

<170>  PatentIn version 3.5

<210>  1
<211>  543
<212>  PRT
<213>  Salmonella typhimurium

<400>  1

Met Leu Lys Tyr Glu Glu Arg Lys Leu Asn Asn Leu Thr Leu Ser Ser
1               5                   10                  15

Phe Ser Lys Val Gly Val Ser Asn Asp Ala Arg Leu Tyr Ile Ala Lys
            20                  25                  30

Glu Asn Thr Asp Lys Ala Tyr Val Ala Pro Glu Lys Phe Ser Ser Lys
        35                  40                  45

Val Leu Thr Trp Leu Gly Lys Met Pro Leu Phe Lys Asn Thr Glu Val
    50                  55                  60

Val Gln Lys His Thr Glu Asn Ile Arg Val Gln Asp Gln Lys Ile Leu
65                  70                  75                  80

Gln Thr Phe Leu His Ala Leu Thr Glu Lys Tyr Gly Glu Thr Ala Val
                85                  90                  95

Asn Asp Ala Leu Leu Met Ser Arg Ile Asn Met Asn Lys Pro Leu Thr
            100                 105                 110

Gln Arg Leu Ala Val Gln Ile Thr Glu Cys Val Lys Ala Ala Asp Glu
        115                 120                 125

Gly Phe Ile Asn Leu Ile Lys Ser Lys Asp Asn Val Gly Val Arg Asn
    130                 135                 140

Ala Ala Leu Val Ile Lys Gly Gly Asp Thr Lys Val Ala Glu Lys Asn
145                 150                 155                 160

Asn Asp Val Gly Ala Glu Ser Lys Gln Pro Leu Leu Asp Ile Ala Leu
                165                 170                 175

Lys Gly Leu Lys Arg Thr Leu Pro Gln Leu Glu Gln Met Asp Gly Asn
        180                 185                 190

Ser Leu Arg Glu Asn Phe Gln Glu Met Ala Ser Gly Asn Gly Pro Leu
    195                 200                 205

Arg Ser Leu Met Thr Asn Leu Gln Asn Leu Asn Lys Ile Pro Glu Ala
210                 215                 220

Lys Gln Leu Asn Asp Tyr Val Thr Thr Leu Thr Asn Ile Gln Val Gly
225                 230                 235                 240

Val Ala Arg Phe Ser Gln Trp Gly Thr Cys Gly Gly Glu Val Glu Arg
                245                 250                 255

Trp Val Asp Lys Ala Ser Thr His Glu Leu Thr Gln Ala Val Lys Lys
            260                 265                 270

Ile His Val Ile Ala Lys Glu Leu Lys Asn Val Thr Ala Glu Leu Glu
        275                 280                 285

Lys Ile Glu Ala Gly Ala Pro Met Pro Gln Thr Met Ser Gly Pro Thr
    290                 295                 300

Leu Gly Leu Ala Arg Phe Ala Val Ser Ser Ile Pro Ile Asn Gln Gln
305                 310                 315                 320

Thr Gln Val Lys Leu Ser Asp Gly Met Pro Val Pro Val Asn Thr Leu
                325                 330                 335

Thr Phe Asp Gly Lys Pro Val Ala Leu Ala Gly Ser Tyr Pro Lys Asn
            340                 345                 350

Thr Pro Asp Ala Leu Glu Ala His Met Lys Met Leu Leu Glu Lys Glu
        355                 360                 365

Cys Ser Cys Leu Val Val Leu Thr Ser Glu Asp Gln Met Gln Ala Lys
    370                 375                 380

Gln Leu Pro Pro Tyr Phe Arg Gly Ser Tyr Thr Phe Gly Glu Val His
385                 390                 395                 400

Thr Asn Ser Gln Lys Val Ser Ser Ala Ser Gln Gly Glu Ala Ile Asp
                405                 410                 415

Gln Tyr Asn Met Gln Leu Ser Cys Gly Glu Lys Arg Tyr Thr Ile Pro
            420                 425                 430

Val Leu His Val Lys Asn Trp Pro Asp His Gln Pro Leu Pro Ser Thr
        435                 440                 445

Asp Gln Leu Glu Tyr Leu Ala Asp Arg Val Lys Asn Ser Asn Gln Asn
    450                 455                 460

Gly Ala Pro Gly Arg Ser Ser Ser Asp Lys His Leu Pro Met Ile His
465                 470                 475                 480

Cys Leu Gly Gly Val Gly Arg Thr Gly Thr Met Ala Ala Ala Leu Val
                485                 490                 495

Leu Lys Asp Asn Pro His Ser Asn Leu Glu Gln Val Arg Ala Asp Phe
            500                 505                 510

Arg Asp Ser Arg Asn Asn Arg Met Leu Glu Asp Ala Ser Gln Phe Val
        515                 520                 525

Gln Leu Lys Ala Met Gln Ala Gln Leu Leu Met Thr Thr Ala Ser
    530                 535                 540

<210>  2
<211>  165
<212>  PRT
<213>  Salmonella typhimurium

<400>  2

Met Leu Lys Tyr Glu Glu Arg Lys Leu Asn Asn Leu Thr Leu Ser Ser
1               5                   10                  15

Phe Ser Lys Val Gly Val Ser Asn Asp Ala Arg Leu Tyr Ile Ala Lys
            20                  25                  30

Glu Asn Thr Asp Lys Ala Tyr Val Ala Pro Glu Lys Phe Ser Ser Lys
        35                  40                  45

Val Leu Thr Trp Leu Gly Lys Met Pro Leu Phe Lys Asn Thr Glu Val
    50                  55                  60

Val Gln Lys His Thr Glu Asn Ile Arg Val Gln Asp Gln Lys Ile Leu
65                  70                  75                  80

Gln Thr Phe Leu His Ala Leu Thr Glu Lys Tyr Gly Glu Thr Ala Val
                85                  90                  95

Asn Asp Ala Leu Leu Met Ser Arg Ile Asn Met Asn Lys Pro Leu Thr
            100                 105                 110

Gln Arg Leu Ala Val Gln Ile Thr Glu Cys Val Lys Ala Ala Asp Glu
        115                 120                 125

Gly Phe Ile Asn Leu Ile Lys Ser Lys Asp Asn Val Gly Val Arg Asn
    130                 135                 140

Ala Ala Leu Val Ile Lys Gly Gly Asp Thr Lys Val Ala Glu Lys Asn
145                 150                 155                 160

Asn Asp Val Gly Ala
                165

<210>  3
<211>  5785
<212>  DNA
<213>  Artificial Sequence

<220>
<223>  Expression vector

<400>  3
taaagcggcc gctgattggc atcaaataaa acgaaaggct cagtcgaaag actgggactt      60

tcgttttatc tgttgtttgt cggtgaacgc tctcctgagt aggacaaatc cgccgcccta     120

gaccgatgca taatgtgcct gtcaaatgga cgaagcaggg attctgcaaa ccctatgcta     180

ctccgtcaag ccgtcaattg tctgattcgt taccaattat gacaacttga cggctacatc     240

attcactttt tcttcacaac cggcacggaa ctcgctcggg ctggccccgg tgcatttttt     300

aaatacccgc gagaaataga gttgatcgtc aaaaccaaca ttgcgaccga cggtggcgat     360

aggcatccgg gtggtgctca aaagcagctt cgcctggctg atacgttggt cctcgcgcca     420

gcttaagacg ctaatcccta actgctggcg gaaaagatgt gacagacgcg acggcgacaa     480

gcaaacatgc tgtgcgacgc tggcgatatc aaaattgctg tctgccaggt gatcgctgat     540

gtactgacaa gcctcgcgta cccgattatc catcggtgga tggagcgact cgttaatcgc     600

ttccatgcgc cgcagtaaca attgctcaag cagatttatc gccagcagct ccgaatagcg     660

cccttcccct tgcccggcgt taatgatttg cccaaacagg tcgctgaaat gcggctggtg     720

cgcttcatcc gggcgaaaga accccgtatt ggcaaatatt gacggccagt taagccattc     780

atgccagtag gcgcgcggac gaaagtaaac ccactggtga taccattcgc gagcctccgg     840

atgacgaccg tagtgatgaa tctctcctgg cgggaacagc aaaatatcac ccggtcggca     900

aacaaattct cgtccctgat ttttcaccac cccctgaccg cgaatggtga gattgagaat     960

ataacctttc attcccagcg gtcggtcgat aaaaaaatcg agataaccgt tggcctcaat    1020

cggcgttaaa cccgccacca gatgggcatt aaacgagtat cccggcagca ggggatcatt    1080

ttgcgcttca gccatacttt tcatactccc gccattcaga gaagaaacca attgtccata    1140

ttgcatcaga cattgccgtc actgcgtctt ttactggctc ttctcgctaa ccaaaccggt 1200

aaccccgctt attaaaagca ttctgtaaca aagcgggacc aaagccatga caaaaacgcg 1260

taacaaaagt gtctataatc acggcagaaa agtccacatt gattatttgc acggcgtcac 1320

actttgctat gccatagcat ttttatccat aagattagcg gatcctacct gacgcttttt 1380

atcgcaactc tctactgttt ctccataccc gtttttttgg gctagcgaat tcgagctcgg 1440

tacccgagcc cgtagaatat gacattaagc tcataataaa agctcaacct gaccgttagt 1500

actaacagca gaattactga aacagtagat tctatcctaa cgacttgtat tagttattat 1560

aacttttcac cctgtaagag aatacactat tatcatgcca cattttaatc ctgttcctgt 1620

atcgaataaa aaattcgtct ttgatgattt catactcaac atggacggct ccctgctacg 1680

ctcagaaaag aaagtcaata ttccgccaaa agaatatgcc gttctggtca tcctgctcga 1740

agccgccggc gagattgtga gtaaaaacac cttactggac caggtatggg gcgacgcgga 1800

agttaacgaa gaatctctta cccgctgtat ttatgcctta cgacgtattc tgtcggaaga 1860

taaagagcat cgttacattg aaacactgta cggacagggc tatcggttta atcgtccggt 1920

cgtagtggtg tctccgccag cgccgcaacc tacgactcat acattggcga tacttccttt 1980

tcagatgcag gatcaggttc aatccgagag tctgcattac tctatcgtga agggattatc 2040

gcagtatgcg ccctttggcc tgagcgtgct gccggtgacc attacgaaga actgccgcag 2100

tgttaaggat attcttgagc tcatggatca attacgcccc gattattata tctccgggca 2160

gatgataccc gatggtaatg ataatattgt acagattgag atagttcggg ttaaaggtta 2220

tcacctgctg caccaggaaa gcattaagtt gatagaacac caacccgctt ctctcttgca 2280

aaacaaaatt gcgaatcttt tgctcagatg tattcccgga cttcgctggg acacaaagca 2340

gattagcgag ctaaattcga ttgacagtac tatggtttac ttacgcggta agcatgagtt 2400

aaatcaatac accccctata gcttacagca agcgcttaaa ttgctgactc aatgcgttaa 2460

catgtcgcca aacagcattg cgccttactg tgcgctggca gaatgctacc tcagcatggc 2520

gcaaatgggg atttttgata aacaaaacgc tatgatcaaa gctaaagaac atgcgattaa 2580

ggcgacagag ctggaccaca ataatccaca agctttagga ttactggggc taattaatac 2640

gattcactca gaatacatcg tcgggagttt gctattcaaa caagctaact tactttcgcc 2700

catttctgca gatattaaat attattatgg ctggaatctt ttcatggctg gtcagttgga 2760

ggaggcctta caaacgatta acgagtgttt aaaattggac ccaacgcgcg cagccgcagg 2820

gatcactaag ctgtggatta cctattatca taccggtatt gatgatgcta tacgtttagg 2880

cgatgaatta cgctcacaac acctgcagga taatccaata ttattaagta tgcaggttat 2940

gtttctttcg cttaaaggta aacatgaact ggcacgaaaa ttaactaaag aaatatccac 3000

gcaggaaata acaggactta ttgctgttaa tcttctttac gctgaatatt gtcagaatag 3060

tgagcgtgcc ttaccgacga taagagaatt tctggaaagt gaacagcgta tagataataa 3120

tccgggatta ttaccgttag tgctggttgc ccacggcgaa gctattgccg agaaaatgtg 3180

gaataaattt aaaaacgaag acaatatttg gttcaaaaga tggaaacagg atccccgctt 3240

gattaaatta cggaatctga gagaggagat atgcattatt tttttatcat cgtaatctgg 3300

ttgcttagca taaatacggc atgggctgat tgctggcttc aggctgaaaa aatgttcaat 3360

attgaatccg aactacttta cgctatcgcc cagcaggaat cggcgatgaa acctggcgcc 3420

attggtcata accgagatgg ttcaaccgat cttggcctga tgcaaattaa cagcttccat 3480

atgaaaaggc tgaaaaaaat ggggattagt gaaaaacagt tgttacagga cccctgcatt 3540

tctgtcattg tgggcgcctc cattttatca gatatgatga aaatctacgg ttatagctgg 3600

gaggccgttg gcgcttataa tgccgggacg tcgccgaaac gatcgaattc gagctcggta 3660

cccgggggga tcctctagag tcgacctgca ggcatgcaag cttggctgtt ttggcggatg 3720

agagaagatt ttcagcctga tacagattaa atcagaacgc agaagcggtc tgataaaaca 3780

gaatttgcct ggcggcagta gcgcggtggt cccacctgac cccatgccga actcagaagt 3840

gaaacgccgt agcgccgatg gtagtgtggg gtctccccat gcgagagtag ggaactgcca 3900

ggcatcaaat aaaacgaaag gctcagtcga aagactgggc ctttcgtttt atctgttgtt 3960

tgtcggtgaa cgctctcctg agtctagggc gttcggctgc ggcgagcggt atcagctcac 4020

tcaaaggcgg taatacggtt atccacagaa tcaggggata acgcaggaaa gaacatgtga 4080

gcaaaaggcc agcaaaaggc caggaaccgt aaaaaggccg cgttgctggc gtttttccat 4140

aggctccgcc cccctgacga gcatcacaaa aatcgacgct caagtcagag gtggcgaaac 4200

ccgacaggac tataaagata ccaggcgttt ccccctggaa gctccctcgt gcgctctcct 4260

gttccgaccc tgccgcttac cggatacctg tccgcctttc tcccttcggg aagcgtggcg 4320

ctttctcata gctcacgctg taggtatctc agttcggtgt aggtcgttcg ctccaagctg 4380

ggctgtgtgc acgaaccccc cgttcagccc gaccgctgcg ccttatccgg taactatcgt 4440

cttgagtcca acccggtaag acacgactta tcgccactgg cagcagccac tggtaacagg 4500

attagcagag cgaggtatgt aggcggtgct acagagttct tgaagtggtg gcctaactac 4560

ggctacacta gaaggacagt atttggtatc tgcgctctgc tgaagccagt taccttcgga 4620

aaaagagttg gtagctcttg atccggcaaa caaaccaccg ctggtagcgg tggttttttt 4680

gtttgcaagc agcagattac gcgcagaaaa aaaggatctc aagaagatcc tttgatcttt 4740

tctacggggt ctgacgctca gtggaacgaa aactcacgtt aagggatttt ggtcatgact 4800

agtgcttgga ttctcaccaa taaaaaacgc ccggcggcaa ccgagcgttc tgaacaaatc 4860

cagatggagt tctgaggtca ttactggatc tatcaacagg agtccaagcg agctcgatat 4920

caaattacgc cccgccctgc cactcatcgc agtactgttg taattcatta agcattctgc 4980

cgacatggaa gccatcacaa acggcatgat gaacctgaat cgccagcggc atcagcacct 5040

tgtcgccttg cgtataatat ttgcccatgg tgaaaacggg ggcgaagaag ttgtccatat 5100

tggccacgtt taaatcaaaa ctggtgaaac tcacccaggg attggctgag acgaaaaaca 5160

tattctcaat aaacccttta gggaaatagg ccaggttttc accgtaacac gccacatctt 5220

gcgaatatat gtgtagaaac tgccggaaat cgtcgtggta ttcactccag agcgatgaaa 5280

acgtttcagt ttgctcatgg aaaacggtgt aacaagggtg aacactatcc catatcacca 5340

gctcaccgtc tttcattgcc atacgaaatt ccggatgagc attcatcagg cgggcaagaa 5400

tgtgaataaa ggccggataa aacttgtgct tatttttctt tacggtcttt aaaaaggccg 5460

taatatccag ctgaacggtc tggttatagg tacattgagc aactgactga aatgcctcaa 5520

aatgttcttt acgatgccat tgggatatat caacggtggt atatccagtg atttttttct 5580

ccattttagc ttccttagct cctgaaaatc tcgataactc aaaaaatacg cccggtagtg 5640

atcttatttc attatggtga aagttggaac ctcttacgtg ccgatcaacg tctcattttc 5700

gccagatatc gacgtctaag aaaccattat tatcatgaca ttaacctata aaaataggcg 5760

tatcacgagg ccctttcgtc ttcac 5785

<210> 4
<211> 114
<212> PRT
<213> Artificial Sequence

<220>
<223> Nanobody

<400> 4

Met Gln Val Gln Leu Val Glu Ser Gly Gly Ala Leu Val Gln Pro Gly
1               5                   10                  15

Gly Ser Leu Arg Leu Ser Cys Ala Ala Ser Gly Phe Pro Val Asn Arg
            20                  25                  30

Tyr Ser Met Arg Trp Tyr Arg Gln Ala Pro Gly Lys Glu Arg Glu Trp
        35                  40                  45

Val Ala Gly Met Ser Ser Ala Gly Asp Arg Ser Ser Tyr Glu Asp Ser
    50                  55                  60

Val Lys Gly Arg Phe Thr Ile Ser Arg Asp Asp Ala Arg Asn Thr Val
65                  70                  75                  80

Tyr Leu Gln Met Asn Ser Leu Lys Pro Glu Asp Thr Ala Val Tyr Tyr

85                  90                  95

Cys Asn Val Asn Val Gly Phe Glu Tyr Trp Gly Gln Gly Thr Gln Val
            100                 105                 110

Thr Val

<210>  5
<211>  123
<212>  PRT
<213>  Artificial Sequence

<220>
<223>  Monobody

<400>  5

Gly Ser Pro Ser Asn Tyr Ile Thr Pro Val Asn Ser Leu Glu Lys His
1               5                   10                  15

Ser Trp Tyr His Gly Pro Val Ser Arg Asn Ala Ala Glu Tyr Leu Leu
            20                  25                  30

Ser Ser Gly Ile Asn Gly Ser Phe Leu Val Arg Glu Ser Glu Ser Ser
        35                  40                  45

Pro Gly Gln Arg Ser Ile Ser Leu Arg Tyr Glu Gly Arg Val Tyr His
    50                  55                  60

Tyr Arg Ile Asn Thr Ala Ser Asp Gly Lys Leu Tyr Val Ser Ser Glu
65                  70                  75                  80

Ser Arg Phe Asn Thr Leu Ala Glu Leu Val His His His Ser Thr Val
                85                  90                  95

Ala Asp Gly Leu Ile Thr Thr Leu His Tyr Pro Ala Pro Lys Arg Asn
            100                 105                 110

Lys Pro Thr Val Tyr Gly Val Ser Pro Asn Tyr
        115                 120

<210>  6
<211>  112
<212>  PRT
<213>  Artificial Sequence

<220>
<223>  Monobody

<400>  6

Ser Leu Glu Lys His Ser Trp Tyr His Gly Pro Val Ser Arg Asn Ala
1               5                   10                  15

Ala Glu Tyr Leu Leu Ser Ser Gly Ile Asn Gly Ser Phe Leu Val Arg
            20                  25                  30

Glu Ser Glu Ser Ser Pro Gly Gln Arg Ser Ile Ser Leu Arg Tyr Glu
        35                  40                  45

Gly Arg Val Tyr His Tyr Arg Ile Asn Thr Ala Ser Asp Gly Lys Leu
    50                  55                  60

Tyr Val Ser Ser Glu Ser Arg Phe Asn Thr Leu Ala Glu Leu Val His
65                  70                  75                  80

His His Ser Thr Val Ala Asp Gly Leu Ile Thr Thr Leu His Tyr Pro
                85                  90                  95

Ala Pro Lys Arg Asn Lys Pro Thr Val Tyr Gly Val Ser Pro Asn Tyr
            100                 105                 110

<210>  7
<211>  7324
<212>  DNA
<213>  Artificial Sequence

<220>
<223>  Expression vector

<400>  7
ctcgagccac aagaaaacga ggtacggcat tgagccgcgt aaggcagtag cgatgtattc      60

attgggcgtt ttttgaatgt tcactaacca ccgtcggggt ttaataactg catcagataa     120

acgcagtcgt taagttctac aaagtcggtg acagataaca ggagtaagta atgcaagcac     180

accaggatat tatcgctaat attggtgaga aattgggttt accgctcact tttgacgaca     240

acaatcagtg cttattatta ctcgatagcg atatttttac gtctattgaa gctaaagatg     300

atatctggtt attgaacggt atgattatac cgttatcgcc tgtttgtggc gattctatct     360

ggcggcagat tatggtgatt aatggtgaac tggctgcgaa taatgaaggt acgttagcgt     420

atattgatgc cgcagagacg ttgttgctta tacatgcaat taccgatctg acaaatactt     480

accatattat atcgcagctt gagtcatttg tgaatcagca ggaagcgctc aaaaacatac     540

tgcaggaata tgctaaagta tgaggagaga aaattgaata atttaacgtt gtcttcgttt     600

tcaaaagttg gtgtgtcgaa tgatgcccga ctttatattg ctaaggaaaa tactgataag     660

gcatatgttg cgcctgaaaa attttcgtca aaagtattaa cctggcttgg aaaaatgccg     720

ttatttaaaa acactgaagt ggtgcaaaaa catacggaaa atatcagagt acaggaccaa     780

aagattttac agacatttct ccatgcacta acggaaaaat atggggaaac agcggttaat 840
gacgcactgt taatgtcccg tataaatatg aacaaacccc ttacccaacg tttagcagtg 900
cagatcacgg acctgggcaa aaaattgctt gaagccgcac gcgcagggca ggatgacgag 960
gtgcgcatcc ttatggcaaa tggggctgat gtcaatgccg cagatgatgt cggcgttact 1020
ccccttcatt tggctgcgca acgcggccac ttggagatcg tagaggtctt attaaagtat 1080
ggtgccgacg tgaatgctgc agacctttgg ggtcagacac cgttacattt ggcggccaca 1140
gcggggcact tggagatcgt ggaggtactg cttaagaacg gcgcggatgt caacgcacgg 1200
gataatatcg ggcacacccc cttgcacttg gctgcctggg caggccacct tgagattgta 1260
gaggtccttt tgaagtatgg cgctgacgtg aatgctcagg ataagtttgg caagacaccc 1320
tttgaccttg ccatcgataa tggtaacgag gatattgctg aagtcctgca aaaggcagcc 1380
gtcgacgatt ataaagatca tgatggcgat tataaagatc atgatattga ttataaagat 1440
gatgatgata aataataatc tagagattat aaagatgacg atgacaagga ttataaagat 1500
gacgatgaca aggattataa agatgacgat gacaagtaat aaagcggccg ctgattggca 1560
tcaaataaaa cgaaaggctc agtcgaaaga ctgggacttt cgttttatct gttgtttgtc 1620
ggtgaacgct ctcctgagta ggacaaatcc gccgccctag accgatgcat aatgtgcctg 1680
tcaaatggac gaagcaggga ttctgcaaac cctatgctac tccgtcaagc cgtcaattgt 1740
ctgattcgtt accaattatg acaacttgac ggctacatca ttcacttttt cttcacaacc 1800
ggcacggaac tcgctcgggc tggccccggt gcatttttta aatacccgcg agaaatagag 1860
ttgatcgtca aaaccaacat tgcgaccgac ggtggcgata ggcatccggg tggtgctcaa 1920
aagcagcttc gcctggctga tacgttggtc ctcgcgccag cttaagacgc taatccctaa 1980
ctgctggcgg aaaagatgtg acagacgcga cggcgacaag caaacatgct gtgcgacgct 2040
ggcgatatca aaattgctgt ctgccaggtg atcgctgatg tactgacaag cctcgcgtac 2100
ccgattatcc atcggtggat ggagcgactc gttaatcgct tccatgcgcc gcagtaacaa 2160
ttgctcaagc agatttatcg ccagcagctc cgaatagcgc ccttcccctt gcccggcgtt 2220
aatgatttgc ccaaacaggt cgctgaaatg cggctggtgc gcttcatccg ggcgaaagaa 2280
ccccgtattg gcaaatattg acggccagtt aagccattca tgccagtagg cgcgcggacg 2340
aaagtaaacc cactggtgat accattcgcg agcctccgga tgacgaccgt agtgatgaat 2400
ctctcctggc gggaacagca aaatatcacc cggtcggcaa acaaattctc gtccctgatt 2460
tttcaccacc ccctgaccgc gaatggtgag attgagaata taacctttca ttcccagcgg 2520
tcggtcgata aaaaaatcga gataaccgtt ggcctcaatc ggcgttaaac ccgccaccag 2580
atgggcatta aacgagtatc ccggcagcag gggatcattt tgcgcttcag ccatactttt 2640
catactcccg ccattcagag aagaaaccaa ttgtccatat tgcatcagac attgccgtca 2700

ctgcgtcttt tactggctct tctcgctaac caaaccggta accccgctta ttaaaagcat 2760

tctgtaacaa agcgggacca aagccatgac aaaaacgcgt aacaaaagtg tctataatca 2820

cggcagaaaa gtccacattg attatttgca cggcgtcaca ctttgctatg ccatagcatt 2880

tttatccata agattagcgg atcctacctg acgcttttta tcgcaactct ctactgtttc 2940

tccatacccg tttttttggg ctagcgaatt cgagctcggt acccgagccc gtagaatatg 3000

acattaagct cataataaaa gctcaacctg accgttagta ctaacagcag aattactgaa 3060

acagtagatt ctatcctaac gacttgtatt agttattata acttttcacc ctgtaagaga 3120

atacactatt atcatgccac attttaatcc tgttcctgta tcgaataaaa aattcgtctt 3180

tgatgatttc atactcaaca tggacggctc cctgctacgc tcagaaaaga aagtcaatat 3240

tccgccaaaa gaatatgccg ttctggtcat cctgctcgaa gccgccggcg agattgtgag 3300

taaaaacacc ttactggacc aggtatgggg cgacgcggaa gttaacgaag aatctcttac 3360

ccgctgtatt tatgccttac gacgtattct gtcggaagat aaagagcatc gttacattga 3420

aacactgtac ggacagggct atcggtttaa tcgtccggtc gtagtggtgt ctccgccagc 3480

gccgcaacct acgactcata cattggcgat acttcctttt cagatgcagg atcaggttca 3540

atccgagagt ctgcattact ctatcgtgaa gggattatcg cagtatgcgc cctttggcct 3600

gagcgtgctg ccggtgacca ttacgaagaa ctgccgcagt gttaaggata ttcttgagct 3660

catggatcaa ttacgccccg attattatat ctccgggcag atgatacccg atggtaatga 3720

taatattgta cagattgaga tagttcgggt taaaggttat cacctgctgc accaggaaag 3780

cattaagttg atagaacacc aacccgcttc tctcttgcaa aacaaaattg cgaatctttt 3840

gctcagatgt attcccggac ttcgctggga cacaaagcag attagcgagc taaattcgat 3900

tgacagtact atggtttact tacgcggtaa gcatgagtta aatcaataca ccccctatag 3960

cttacagcaa gcgcttaaat tgctgactca atgcgttaac atgtcgccaa acagcattgc 4020

gccttactgt gcgctggcag aatgctacct cagcatggcg caaatgggga tttttgataa 4080

acaaaacgct atgatcaaag ctaaagaaca tgcgattaag gcgacagagc tggaccacaa 4140

taatccacaa gctttaggat tactggggct aattaatacg attcactcag aatacatcgt 4200

cgggagtttg ctattcaaac aagctaactt actttcgccc atttctgcag atattaaata 4260

ttattatggc tggaatcttt tcatggctgg tcagttggag gaggccttac aaacgattaa 4320

cgagtgttta aaattggacc caacgcgcgc agccgcaggg atcactaagc tgtggattac 4380

ctattatcat accggtattg atgatgctat acgtttaggc gatgaattac gctcacaaca 4440

cctgcaggat aatccaatat tattaagtat gcaggttatg tttctttcgc ttaaaggtaa 4500

acatgaactg gcacgaaaat taactaaaga aatatccacg caggaaataa caggacttat 4560

tgctgttaat cttctttacg ctgaatattg tcagaatagt gagcgtgcct taccgacgat 4620

aagagaattt ctggaaagtg aacagcgtat agataataat ccgggattat taccgttagt 4680

gctggttgcc cacggcgaag ctattgccga gaaaatgtgg aataaattta aaaacgaaga 4740

caatatttgg ttcaaaagat ggaaacagga tccccgcttg attaaattac ggaatctgag 4800

agaggagata tgcattattt ttttatcatc gtaatctggt tgcttagcat aaatacggca 4860

tgggctgatt gctggcttca ggctgaaaaa atgttcaata ttgaatccga actactttac 4920

gctatcgccc agcaggaatc ggcgatgaaa cctggcgcca ttggtcataa ccgagatggt 4980

tcaaccgatc ttggcctgat gcaaattaac agcttccata tgaaaaggct gaaaaaaatg 5040

gggattagtg aaaaacagtt gttacaggac ccctgcattt ctgtcattgt gggcgcctcc 5100

attttatcag atatgatgaa aatctacggt tatagctggg aggccgttgg cgcttataat 5160

gccgggacgt cgccgaaacg atcgaattcg agctcggtac ccggggggat cctctagagt 5220

cgacctgcag gcatgcaagc ttggctgttt tggcggatga gagaagattt tcagcctgat 5280

acagattaaa tcagaacgca gaagcggtct gataaaacag aatttgcctg gcggcagtag 5340

cgcggtggtc ccacctgacc ccatgccgaa ctcagaagtg aaacgccgta gcgccgatgg 5400

tagtgtgggg tctccccatg cgagagtagg gaactgccag gcatcaaata aaacgaaagg 5460

ctcagtcgaa agactgggcc tttcgtttta tctgttgttt gtcggtgaac gctctcctga 5520

gtctagggcg ttcggctgcg gcgagcggta tcagctcact caaaggcggt aatacggtta 5580

tccacagaat caggggataa cgcaggaaag aacatgtgag caaaaggcca gcaaaaggcc 5640

aggaaccgta aaaaggccgc gttgctggcg tttttccata ggctccgccc ccctgacgag 5700

catcacaaaa atcgacgctc aagtcagagg tggcgaaacc cgacaggact ataaagatac 5760

caggcgtttc cccctggaag ctccctcgtg cgctctcctg ttccgaccct gccgcttacc 5820

ggatacctgt ccgcctttct cccttcggga agcgtggcgc tttctcatag ctcacgctgt 5880

aggtatctca gttcggtgta ggtcgttcgc tccaagctgg gctgtgtgca cgaacccccc 5940

gttcagcccg accgctgcgc cttatccggt aactatcgtc ttgagtccaa cccggtaaga 6000

cacgacttat cgccactggc agcagccact ggtaacagga ttagcagagc gaggtatgta 6060

ggcggtgcta cagagttctt gaagtggtgg cctaactacg gctacactag aaggacagta 6120

tttggtatct gcgctctgct gaagccagtt accttcggaa aaagagttgg tagctcttga 6180

tccggcaaac aaaccaccgc tggtagcggt ggtttttttg tttgcaagca gcagattacg 6240

cgcagaaaaa aaggatctca agaagatcct ttgatctttt ctacggggtc tgacgctcag 6300

tggaacgaaa actcacgtta agggattttg gtcatgacta gtgcttggat tctcaccaat 6360

aaaaaacgcc cggcggcaac cgagcgttct gaacaaatcc agatggagtt ctgaggtcat 6420

tactggatct atcaacagga gtccaagcga gctcgatatc aaattacgcc ccgccctgcc 6480

actcatcgca gtactgttgt aattcattaa gcattctgcc gacatggaag ccatcacaaa 6540

cggcatgatg aacctgaatc gccagcggca tcagcacctt gtcgccttgc gtataatatt 6600

tgcccatggt gaaaacgggg gcgaagaagt tgtccatatt ggccacgttt aaatcaaaac 6660

tggtgaaact cacccaggga ttggctgaga cgaaaaacat attctcaata aaccctttag 6720

ggaaataggc caggttttca ccgtaacacg ccacatcttg cgaatatatg tgtagaaact 6780

gccggaaatc gtcgtggtat tcactccaga gcgatgaaaa cgtttcagtt tgctcatgga 6840

aaacggtgta acaagggtga acactatccc atatcaccag ctcaccgtct ttcattgcca 6900

tacgaaattc cggatgagca ttcatcaggc gggcaagaat gtgaataaag gccggataaa 6960

acttgtgctt atttttcttt acggtcttta aaaaggccgt aatatccagc tgaacggtct 7020

ggttataggt acattgagca actgactgaa atgcctcaaa atgttcttta cgatgccatt 7080

gggatatatc aacggtggta tatccagtga tttttttctc cattttagct tccttagctc 7140

ctgaaaatct cgataactca aaaaatacgc ccggtagtga tcttatttca ttatggtgaa 7200

agttggaacc tcttacgtgc cgatcaacgt ctcattttcg ccagatatcg acgtctaaga 7260

aaccattatt atcatgacat taacctataa aaataggcgt atcacgaggc cctttcgtct 7320

tcac 7324